(19) Europäisches Patentamt / European Patent Office / Office européen des brevets

(11) **EP 4 445 910 A1**

(12) **EUROPEAN PATENT APPLICATION**
published in accordance with Art. 153(4) EPC

(43) Date of publication:
**16.10.2024  Bulletin 2024/42**

(21) Application number: **22904247.8**

(22) Date of filing: **07.12.2022**

(51) International Patent Classification (IPC):
*A61K 39/00* (2006.01)    *A61K 47/55* (2017.01)
*A61P 1/04* (2006.01)    *A61P 3/10* (2006.01)
*A61P 9/00* (2006.01)    *A61P 9/10* (2006.01)
*A61P 17/00* (2006.01)    *A61P 17/06* (2006.01)
*A61P 19/00* (2006.01)    *A61P 19/02* (2006.01)
*A61P 25/00* (2006.01)    *A61P 25/28* (2006.01)
*A61P 27/02* (2006.01)    *A61P 29/00* (2006.01)
*A61P 35/00* (2006.01)    *A61P 37/00* (2006.01)
*A61P 37/02* (2006.01)    *A61P 37/04* (2006.01)
*A61P 37/06* (2006.01)    *A61P 37/08* (2006.01)
*A61P 43/00* (2006.01)

(52) Cooperative Patent Classification (CPC):
**A61K 39/00; A61K 47/55; A61P 1/04; A61P 3/10;
A61P 9/00; A61P 9/10; A61P 17/00; A61P 17/06;
A61P 19/00; A61P 19/02; A61P 25/00;
A61P 25/28; A61P 27/02; A61P 29/00;
A61P 35/00;**                            (Cont.)

(86) International application number:
**PCT/JP2022/045045**

(87) International publication number:
**WO 2023/106319 (15.06.2023 Gazette 2023/24)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.12.2021  JP 2021199561
07.10.2022  JP 2022162795**

(71) Applicants:
• **OSAKA UNIVERSITY
Suita-shi
Osaka 565-0871 (JP)**
• **Funpep Co., Ltd.
Ibaraki-shi, Osaka 567-0085 (JP)**

(72) Inventors:
• **NAKAGAMI, Hironori
Suita-shi, Osaka 565-0871 (JP)**
• **HAYASHI, Hiroki
Suita-shi, Osaka 565-0871 (JP)**
• **MORISHITA, Ryuichi
Suita-shi, Osaka 565-0871 (JP)**
• **SAKAGUCHI, Makoto
Ibaraki-shi, Osaka 567-0085 (JP)**
• **KAWABATA, Sotaro
Ibaraki-shi, Osaka 567-0085 (JP)**

(74) Representative: **Vossius & Partner
Patentanwälte Rechtsanwälte mbB
Siebertstrasse 3
81675 München (DE)**

Remarks:
The complete document including Reference
Table(s) and the Sequence Listing(s) can be
downloaded from the EPO website

(54) **VACCINE COMPOSITION FOR INDUCING ANTI-IL-23 ANTIBODY**

(57)  The present invention provides a vaccine composition containing a complex of a T cell receptor antigen peptide and a B cell receptor antigen peptide and capable of inducing the production of an antibody against IL-23, wherein the

EP 4 445 910 A1

B cell receptor antigen peptide is represented by the following formula (I):

$$X1\text{-}X2\text{-}X3\text{-}X4\text{-}X5\text{-}X6\text{-}X7\text{-}X8 \qquad (I)$$

wherein
X1 is S, A, G, T, K, or R,
X2 is P, A, G, S, T, K, or R,
X3 is S, A, G, T, K, or R,
X4 is Q, A, G, T, or N,
X5 is P, A, G, S, T, Q, or N,
X6 is W, A, Y, or F,
X7 is Q, A, G, T, or N, and
X8 is R, A, G, or K.

[Fig. 6]

(a) Ear swelling

(b) Inflammatory cytokine mRNA expression

(52) Cooperative Patent Classification (CPC): (Cont.)
   A61P 37/00; A61P 37/02; A61P 37/04; A61P 37/06; A61P 37/08; A61P 43/00; C07K 7/06; C07K 7/08;
   C07K 14/00; C07K 14/54

**Description**

[Technical Field]

The present invention relates to a vaccine composition. More particularly, the present invention relates to a vaccine composition that can induce an antibody against IL-23 in a living body.

[Background Art]

Chronic inflammation is known to be involved in the development of a wide variety of chronic diseases, and it has been reported that IL-23 is the factor to be the starting point for chronic inflammation (Non Patent Literatures 1 and 2).

IL-23 is a heterodimer consisting of two subunit proteins, p19 and p40, and is classified as an inflammatory cytokine. IL-23 is involved in the induction of memory T cell proliferation and also contributes to promotion and stabilization of the differentiation of naive T cells into Th17 cells. Th17 cells differentiated and stabilized by IL-23 secrete multiple types of cytokines and inflammatory effectors, and promote chronic inflammation.

Deregulation of such Th17/IL-23 pathway has been reported to be related to many diseases (some examples are psoriasis, cancer, rheumatoid arthritis, systemic lupus erythematosus, diabetes, atherosclerosis, inflammatory bowel disease, multiple sclerosis, Alzheimer's disease, and the like) (Non Patent Literature 3).

As described above, IL-23 is involved in the development and progression of various diseases through chronic inflammation. Therefore, drug discovery research is being intensively conducted on IL-23 as a therapeutic target. Among others, a comparatively large number of findings for psoriasis are being accumulated.

Psoriasis is a chronic inflammatory skin disease caused by abnormalities in Th17 cells. In psoriasis, Th17 cells are stimulated by IL-23, and the Th17 cells release inflammatory cytokines IL-17 and IL-22, causing an inflammatory response. The prevalence of psoriasis is estimated to be 0.9 to 11.43% (WHO report, 2016). Most patients with psoriasis have mild to moderate psoriasis, but patients with severe psoriasis are accompanied by complications such as serious skin symptoms and psoriatic arthritis, and QOL of those patients is extremely low.

In the treatment of psoriasis, topical ointments such as steroids and vitamin D analogs are used for patients with mild to moderate psoriasis. When standard treatments do not provide a sufficient therapeutic effect, biological medicines such as anti-TNF-$\alpha$ antibody, anti-IL-17 antibody, or anti-IL-23 antibody is administered. In particular, anti-IL-23 antibody drugs have been clinically confirmed to have high efficacy (Ustekinumab targetting p40 subunit (trade name "Stelara" (registered trademark)), Risankizumab targetting p19 subunit (trade name "Skyrizi" (registered trade mark)), Guselkumab (trade name "Tremfya" (registered trade mark)), and Tildrakizumab (trade name "Ilumya" (registered trade mark)), and the like). Risankizumab has been reported to show a PASI90 (almost complete disappearance of psoriasis lesion) rate that is about twice as high as that of Ustekinumab, and thus the molecular target has been shifting from p40 to p19.

However, the production costs of antibody drugs are high because large investments in biomanufacturing facilities are required, and a relatively large amount of antibody needs to be administered in one treatment, resulting in very high drug prices. This not only limits patient access, but also puts pressure on healthcare financing. Therefore, in order to solve the problems with antibody drugs, the development of "peptide vaccines" that can induce the desired antibodies in the body of the treatment target is underway, and peptide vaccines that can induce anti-IL-23 antibody are also considered in the treatment of psoriasis.

For example, it has been reported that a peptide vaccine containing a peptide fragment of the junction site between $\alpha$-loop C and $\alpha$-loop D of the mouse IL-23 p19 subunit is effective in mouse collagen-induced arthritis (CIA) and TNBS-induced colitis models (Non Patent Literatures 4 and 5). In Patent Literature 1, moreover, a detailed study was conducted using various peptide fragments contained in the corresponding site in the human IL-23 p19 subunit, and a B-cell epitope capable of inducing anti-IL-23 neutralizing antibodies was identified. However, since these peptide vaccines are used with the proteins such as keyhole limpet hemocyanin (KLH) and virus-like particle (VLP) as carriers to enhance immunogenicity, problems occur such as production costs and the risk of inducing carrier antibodies, and further improvements are desired. In addition, in order to induce sufficient antibody production, activation of helper T cells and innate immunity is necessary, due to which conventional peptide vaccines are generally used in combination with adjuvants such as incomplete Freund adjuvant (IFA) and aluminum salt (Alum), which poses a risk in safety aspects.

[Citation List]

[Patent Literature]

[Patent Literature 1]
WO2016/193405

[Non Patent Literature]

[Non Patent Literature 1]
Christina H Liu et al., Nat Immunol. 2017 Oct 18; 18(11):1175-1180.
[Non Patent Literature 2]
Yoichiro Iwakura et al., J Clin Invest. 2006 May; 116(5):1218-22.
[Non Patent Literature 3]
Sarah L Gaffen et al., Nat Rev Immunol. 2014 Sep; 14(9):585-600.
[Non Patent Literature 4]
Rojo A Ratsimandresy et al., Vaccine. 2011; 29:9329-9336.
[Non Patent Literature 5]
Qingdong Guan et al., Immunotherapy. 2013; 5:1313-1322.

[Summary of Invention]

[Technical Problem]

With the aforementioned background, the problem of the present invention is to provide a novel peptide vaccine having sufficient antigenicity and capable of efficiently inducing an anti-IL-23 antibody in a living body, even without conjugating a carrier protein or concurrently using an adjuvant.

[Solution to Problem]

The present inventors have conducted intensive studies of the above-mentioned problem and found that a complex of a T cell receptor antigen peptide having a specific amino acid sequence and a B cell receptor antigen peptide having a specific amino acid sequence in IL-23 p19 protein (corresponding to positions 151-158 of human p19; positions 152-159 of mouse p19) or a partially modified sequence thereof can induce anti-IL-23 neutralizing antibodies extremely efficiently in vivo. According to the above-mentioned Patent Literature 1, the peptide vaccine containing the peptide fragment consisting of the amino acid sequence at positions 152 to 159 of human p19, which was shifted just one amino acid toward the C-terminus in the position in human p19 (therefore, 7 out of 8 amino acids are common) did not induce an inhibitory activity of anti-IL-23 function (promotion of IL-17 secretion). Thus, the above-mentioned results are very surprising.

In addition, the present inventors have also confirmed that the symptoms accompanying enteritis are markedly improved by the administration of the peptide vaccine in mouse colitis model.

The present inventors have conducted further studies based on such findings and completed the present invention.

Accordingly, the present invention provides the following.

Item [1]
A vaccine composition comprising a complex of a T cell receptor antigen peptide and a B cell receptor antigen peptide and capable of inducing the production of an antibody against IL-23, wherein the B cell receptor antigen peptide is represented by the following formula (I):

$$X1-X2-X3-X4-X5-X6-X7-X8 \qquad (I)$$

wherein

X1 is S, A, G, T, K, or R,
X2 is P, A, G, S, T, K, or R,
X3 is S, A, G, T, K, or R,
X4 is Q, A, G, T, or N,
X5 is P, A, G, S, T, Q, or N,
X6 is W, A, Y, or F,
X7 is Q, A, G, T, or N, and
X8 is R, A, G, or K.

Item [2]

The vaccine composition of Item [1], wherein the N-terminal amino acid of the complex is acetylated and/or the C-terminal amino acid of the complex is amidated.

Item [3]

The vaccine composition of Item [1] or [2], wherein X6 in the aforementioned B cell receptor antigen peptide is W.

Item [4]

The vaccine composition of any one of Items [1] to [3], wherein X7 in the aforementioned B cell receptor antigen peptide is Q.

Item [5]

The vaccine composition of any one of Items [1] to [4], wherein X8 in the aforementioned B cell receptor antigen peptide is R.

Item [6]

The vaccine composition of any one of Items [1] to [5], wherein the B cell receptor antigen peptide comprises the amino acid sequence shown in any of SEQ ID NOs: 2, 6 - 14 and 17 - 26.

Item [7]

The vaccine composition of any one of Items [1] to [6], wherein the T cell receptor antigen peptide comprises the amino acid sequence shown in SEQ ID NO: 38.

Item [8]

The vaccine composition of any one of Items [1] to [7], wherein, in the complex, the C-terminus of the T cell receptor antigen peptide is bonded to the N-terminus of the B cell receptor antigen peptide.

Item [9]

The vaccine composition of any one of Items [1] to [8], wherein the T cell receptor antigen peptide and the B cell receptor antigen peptide are bonded via a linker.

Item [10]

The vaccine composition of Item [9], wherein the aforementioned linker is an amino acid or a peptide.

Item [11]

The vaccine composition of any one of Items [1] to [10], which does not contain an additive as an adjuvant.

Item [12]

The vaccine composition of any one of Items [1] to [11], for the treatment or prevention of an IL-23-associated disease. Item [13]

The vaccine composition of Item [12], wherein the IL-23-associated disease is selected from the group consisting of psoriasis, psoriatic arthritis, rheumatoid arthritis, systemic lupus erythematosus, diabetes (preferably type I diabetes), atherosclerosis, inflammatory bowel disease (IBD)/Crohn's disease, multiple sclerosis, Behcet's disease, ankylopoietic spondylarthritis, Vogt-Koyanagi-Harada disease, chronic granulomatous disease, hidradenitis suppurativa, Anti-Neutrophil Cytoplasmic Antibody (ANCA) Associated Vasculitis, neurodegenerative disease, atopic dermatitis, graft-versus-host disease, and cancer.

Item [1A]

A method for inducing the production of an antibody against IL-23 in a subject, comprising administering an effective amount of a complex of a T cell receptor antigen peptide and a B cell receptor antigen peptide to the subject, wherein the B cell receptor antigen peptide is represented by the following formula (I):

$$X1-X2-X3-X4-X5-X6-X7-X8 \qquad (I)$$

wherein

X1 is S, A, G, T, K, or R,
X2 is P, A, G, S, T, K, or R,
X3 is S, A, G, T, K, or R,
X4 is Q, A, G, T, or N,
X5 is P, A, G, S, T, Q, or N,
X6 is W, A, Y, or F,
X7 is Q, A, G, T, or N, and
X8 is R, A, G, or K.

Item [1B]

A complex of a T cell receptor antigen peptide and a B cell receptor antigen peptide for use in the induction of production of an antibody against IL-23, wherein the B cell receptor antigen peptide is represented by the following formula (I):

X1-X2-X3-X4-X5-X6-X7-X8　　　　　(I)

wherein

X1 is S, A, G, T, K, or R,
X2 is P, A, G, S, T, K, or R,
X3 is S, A, G, T, K, or R,
X4 is Q, A, G, T, or N,
X5 is P, A, G, S, T, Q, or N,
X6 is W, A, Y, or F,
X7 is Q, A, G, T, or N, and
X8 is R, A, G, or K.

Item [1C]
Use of a complex of a T cell receptor antigen peptide and a B cell receptor antigen peptide in the production of a medicament for the treatment or prevention of an IL-23-associated disease, wherein the B cell receptor antigen peptide is represented by the following formula (I):

X1-X2-X3-X4-X5-X6-X7-X8　　　　　(I)

wherein

X1 is S, A, G, T, K, or R,
X2 is P, A, G, S, T, K, or R,
X3 is S, A, G, T, K, or R,
X4 is Q, A, G, T, or N,
X5 is P, A, G, S, T, Q, or N,
X6 is W, A, Y, or F,
X7 is Q, A, G, T, or N, and
X8 is R, A, G, or K.

Item [14]
A B cell receptor antigen peptide represented by the following formula (I):

X1-X2-X3-X4-X5-X6-X7-X8　　　　　(I)

wherein

X1 is S, A, G, T, K, or R,
X2 is P, A, G, S, T, K, or R,
X3 is S, A, G, T, K, or R,
X4 is Q, A, G, T, or N,
X5 is P, A, G, S, T, Q, or N,
X6 is W, A, Y, or F,
X7 is Q, A, G, T, or N, and
X8 is R, A, G, or K.

Item [15]
The B cell receptor antigen peptide of Item [14], wherein X6 in the aforementioned formula (I) is W.
Item [16]
The B cell receptor antigen peptide of Item [14] or [15], wherein X7 in the aforementioned formula (I) is Q.
Item [17]
The B cell receptor antigen peptide of any of Items [14] to [16], wherein X8 in the aforementioned formula (I) is R.

[Advantageous Effects of Invention]

According to the present invention, production of an anti-IL-23 antibody in a living body can be induced very efficiently without using a carrier protein or an adjuvant. Therefore, it becomes possible to treat and/or prevent diseases related to IL-23 safely at a low cost.

[Brief Description of Drawings]

[Fig. 1]

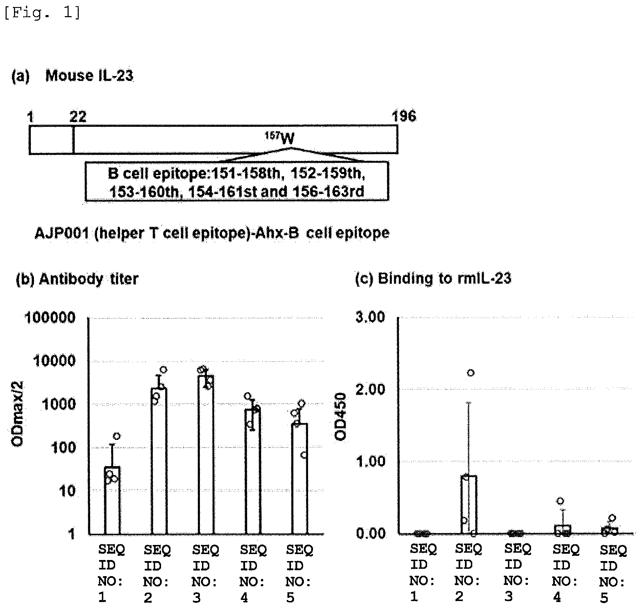

Fig. 1 (a) is a schematic diagram of each AJP001 conjugated peptide used in Example 1. Fig. 1 (b) is a graph showing the antibody titer of antiserum collected from the mice administered with each AJP001 conjugated peptide at 5 weeks after the initial administration of the peptide in Example 1. Fig. 1 (c) is a graph showing the binding ability to rmIL-23 of serum collected from the mice administered with each AJP001 conjugated peptide at 5 weeks after the first administration of the peptide in Example 1.
[Fig. 2]

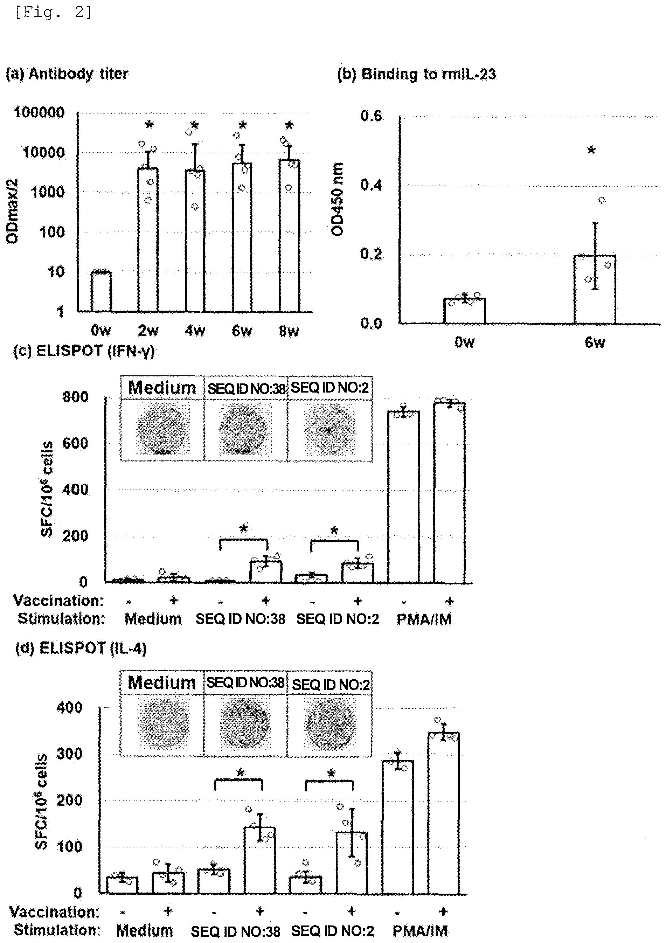

Fig. 2 is a graph showing the results of the mouse immunogenicity evaluation test using AJP001 conjugated peptide (SEQ ID NO: 2). Fig. 2(a) is a graph showing the antibody titer (GMT) of serum collected over time from the mice administered with AJP001 conjugated peptide (SEQ ID NO: 2). Fig. 2(b) is a graph showing the binding ability to rmIL-23 of serum collected from the mice administered with AJP001 conjugated peptide (SEQ ID NO: 2) at 6 weeks after the first administration. Fig. 2(c) and (d) are diagrams showing the results of T cell activation evaluation (the number of IFN-$\gamma$ positive cells and the number of IL-4 positive cells) in the mice administered with AJP001 conjugated peptide (SEQ ID NO: 2).
[Fig. 3]

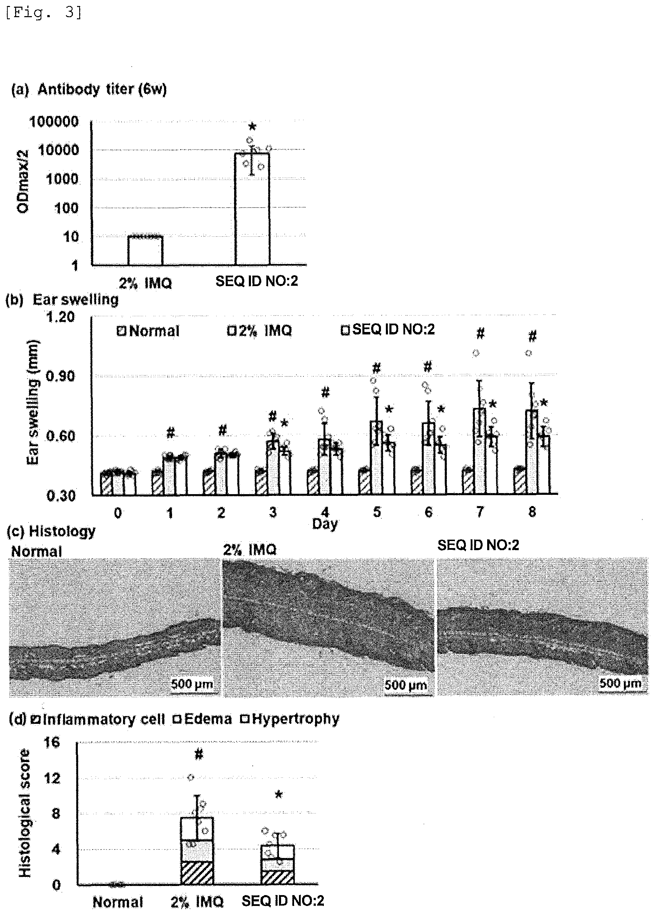

Fig. 3 shows the results of an efficacy evaluation test in mouse psoriasis model in Example 3 using AJP001 conjugated peptide (SEQ ID NO: 2). Fig. 3(a) is a graph showing the antibody titer of serum collected from hairless rats administered with AJP001 conjugated peptide (SEQ ID NO: 2) at 6 weeks after the first administration. Fig. 3(b) is a graph showing the auricular inflammatory response (increase in auricular thickness and inflammatory changes of tissue) over time in the group administered with AJP001 conjugated peptide (SEQ ID NO: 2) and the vehicle control group. Fig. 3(c) shows photographs of HE-stained specimens of the auricle prepared under respective conditions. Fig. 3(d) is a graph showing the results of scoring inflammatory cell infiltration, edema, and thickening by HE-stained specimens of the auricle prepared under respective conditions with a microscope.
[Fig. 4]

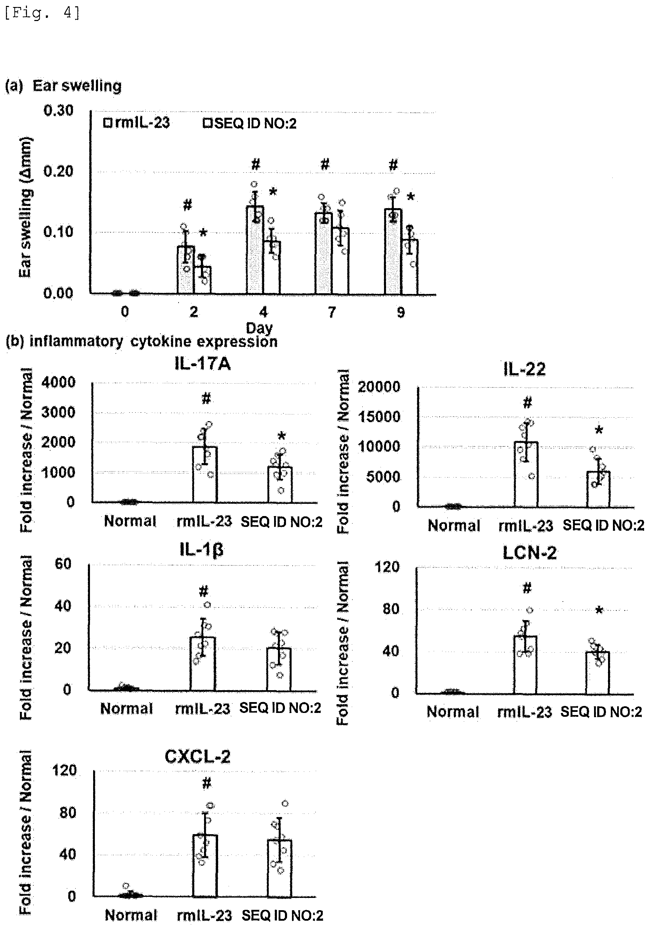

Fig. 4 shows the results of an efficacy evaluation test for IL-23-induced mouse auricle inflammation model in Example 4, using AJP001 conjugated peptide (SEQ ID NO: 2). Fig. 4(a) shows time-course changes in the auricular thickness under each condition. Fig. 4(b) is a graph showing the expression of IL-17A, IL-22, IL-1$\beta$, LCN-2, and CXCL-2 mRNAs in the auricle under each condition.
[Fig. 5]

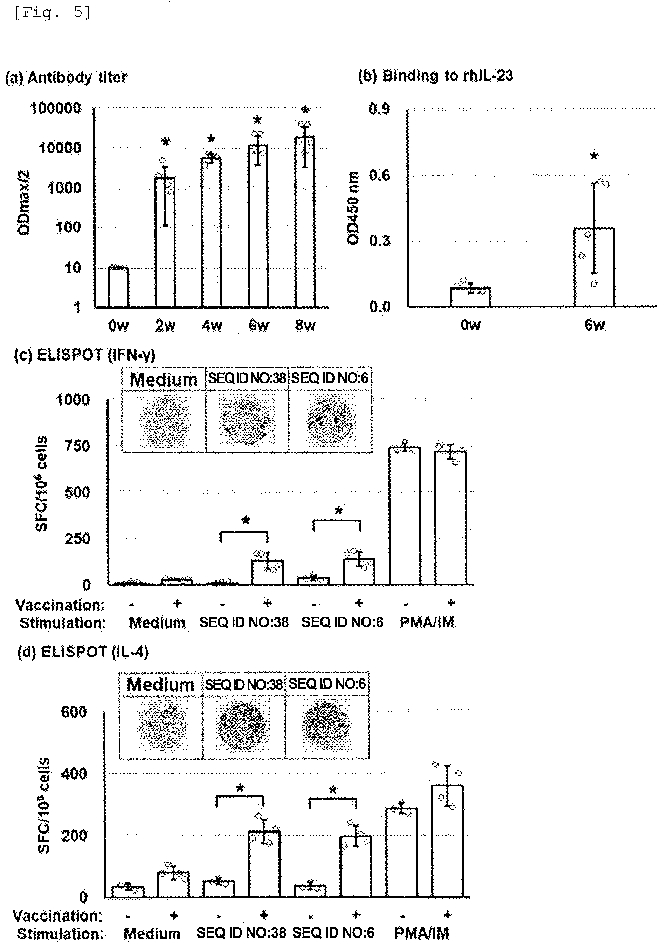

Fig. 5 shows the results of the mouse immunogenicity evaluation test using AJP001 conjugated peptide (SEQ ID NO: 6) in Example 6. Fig. 5(a) is a graph showing the antibody titer (GMT) of serum collected over time from the mice administered with AJP001 conjugated peptide (SEQ ID NO: 6). Fig. 5(b) is a graph showing the binding ability to rhIL-23 of serum collected from the mice administered with AJP001 conjugated peptide (SEQ ID NO: 6) at 6 weeks after the first administration. Figs. 5(c) and (d) shows the results of T cell activation evaluation (the number of IFN-$\gamma$ positive cells and the number of IL-4 positive cells) in the mice administered with AJP001 conjugated peptide (SEQ ID NO: 6).
[Fig. 6]

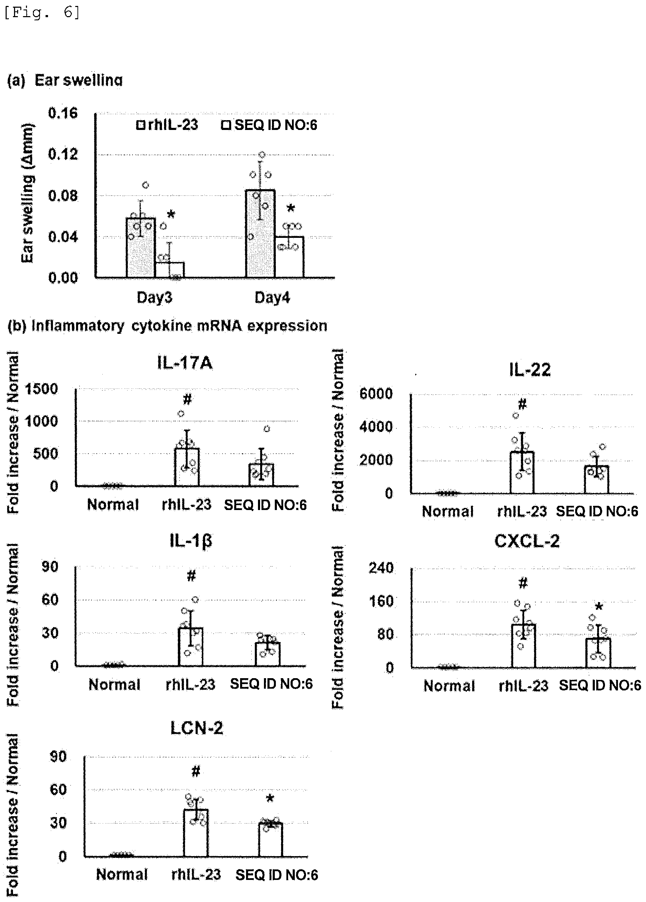

Fig. 6 shows the results of an efficacy evaluation test in IL-23-induced mouse auricle inflammation model using AJP001 conjugated peptide (SEQ ID NO: 6) in Example 7. Fig. 6(a) shows changes in the auricular thickness on day 3 and day 4 under each condition. Fig. 6(b) shows the expression of IL-17A, IL-22, IL-1$\beta$, LCN-2, and CXCL-2 mRNAs in the auricle under each condition.
[Fig. 7]

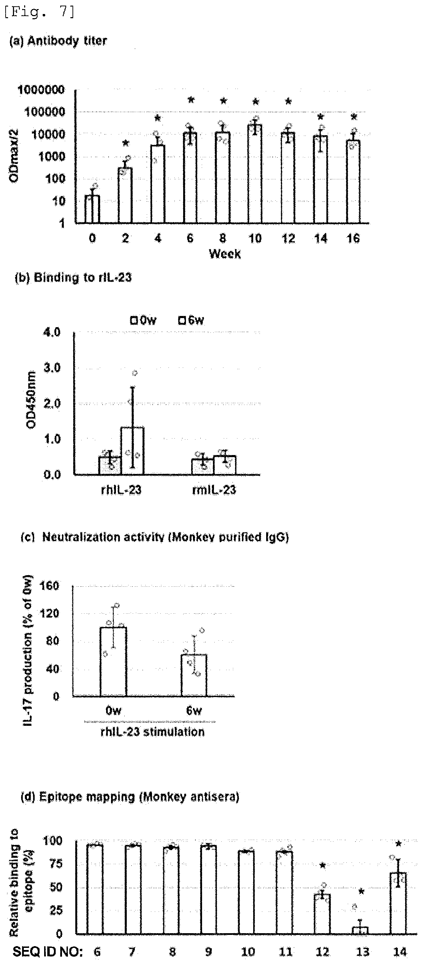

Fig. 7 shows diagrams presenting the results of monkey immunogenicity evaluation test using AJP001 conjugated peptide (SEQ ID NO: 6) in Example 8. Fig. 7(a) is a graph showing the antibody titer (GMT) of serum collected over time from Macaca fascicularis administered with AJP001 conjugated peptide (SEQ ID NO: 6). Fig. 7(b) is a graph showing the binding ability to rhIL-23 and rmIL-23 of serum collected from the Macaca fascicularis administered with AJP001 conjugated peptide (SEQ ID NO: 6) at 6 weeks after the first administration. Fig. 7(c) is a graph showing the evaluation of neutralization activity of IgG derived from Macaca fascicularis administered with AJP001 conjugated peptide (SEQ ID NO: 6). Fig. 7 (d) is a graph showing the results of epitope mapping of anti-IL-23 antibody induced by AJP001 conjugated peptide (SEQ ID NO: 6).
[Fig. 8-1]

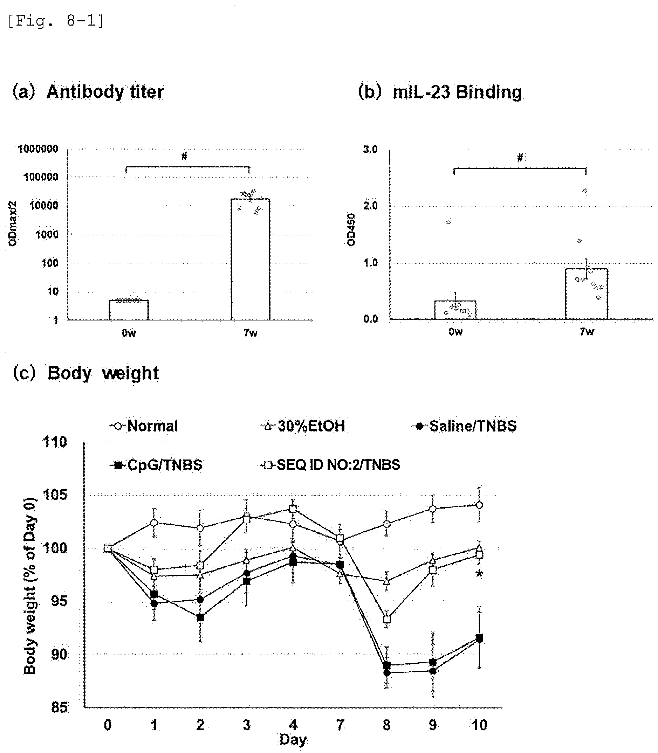

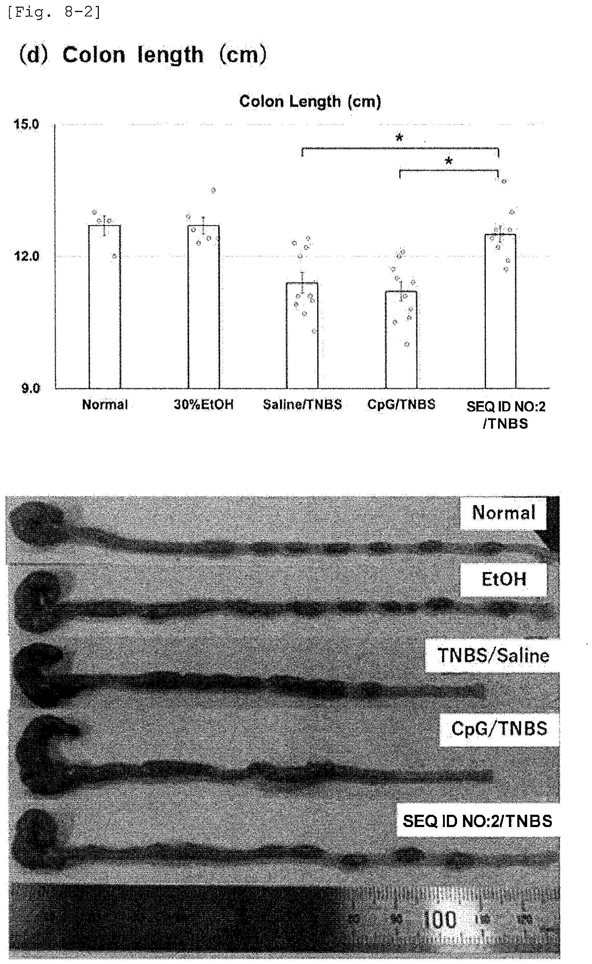

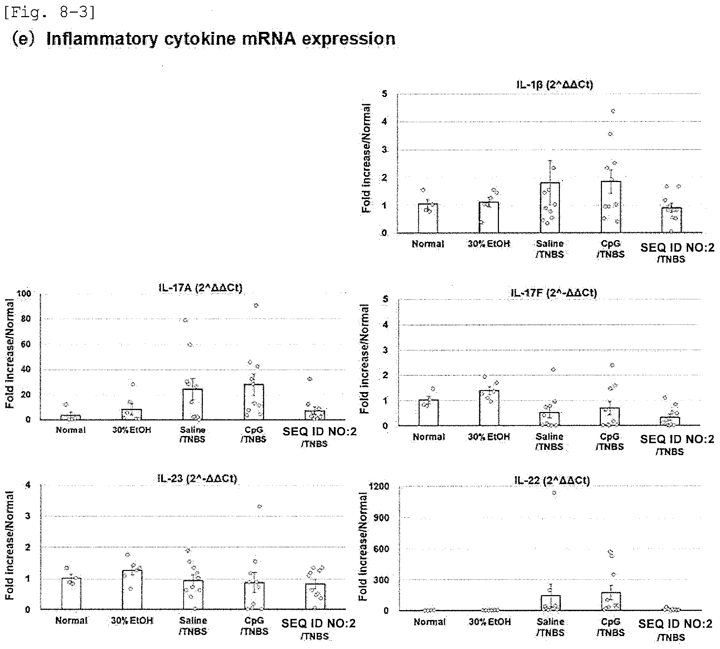

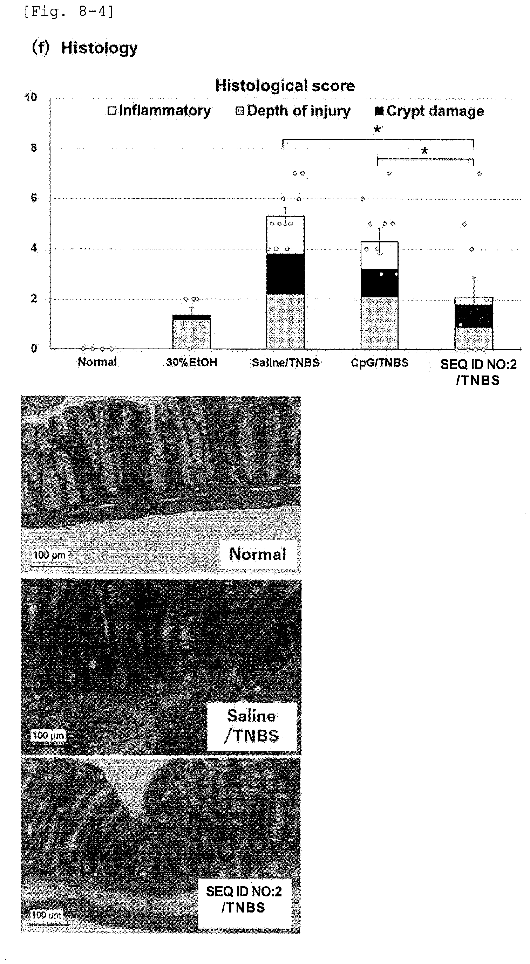

Fig. 8-1 shows an efficacy evaluation test in TNBS-induced mouse colitis model using AJP001 conjugated peptide (SEQ ID NO: 2) in Example 9. Therein, (a) and (b) show the results of antibody titer (GMT) and binding to mIL-23 as measured by ELISA method, with respect to the serum obtained from the mice administered with AJP001 conjugated peptide (SEQ ID NO: 2), followed by induction of colitis with TNBS (on day 10 from TNBS administration as the standard); (c) shows changes in the body weight before and after administration of TNBS in the mice administered with AJP001 conjugated peptide (SEQ ID NO: 2), followed by induction of colitis with TNBS.

[Fig. 8-2]

Fig. 8-2 shows an efficacy evaluation test in TNBS-induced mouse colitis model using AJP001 conjugated peptide (SEQ ID NO: 2) in Example 9. (d) shows a diagram and a photograph presenting the measurement results of the length (cm) of large intestine on day 10 of the mice administered with AJP001 conjugated peptide (SEQ ID NO: 2), followed by induction of colitis with TNBS.

[Fig. 8-3]

Fig. 8-3 shows an efficacy evaluation test in TNBS-induced mouse colitis model using AJP001 conjugated peptide (SEQ ID NO: 2) in Example 9. (e) shows the measurement results of mRNA expression of various cytokines in a large intestinal tissue 2 cm from the anus, on day 10 of the mice administered with AJP001 conjugated peptide (SEQ ID NO: 2), followed by induction of colitis with TNBS.

[Fig. 8-4]

Fig. 8-4 shows an efficacy evaluation test in TNBS-induced mouse colitis model using AJP001 conjugated peptide (SEQ ID NO: 2) in Example 9. (f) shows a diagram and a photograph presenting the results of a pathological test in a large intestinal tissue 2 to 4 cm from the anus, on day 10 of the mice administered with AJP001 conjugated peptide (SEQ ID NO: 2), followed by induction of colitis with TNBS.

[Description of Embodiments]

The present invention is described in detail in the following.

1. Vaccine composition

The present invention provides a vaccine composition containing a complex of a T cell receptor antigen peptide and a B cell receptor antigen peptide and capable of inducing the production of an antibody against IL-23 (therefore, suitable for the treatment or prevention of IL-23-associated diseases) (hereinafter sometimes referred to as "the vaccine composition of the present invention").

The peptide in the present specification is described according to the common practice of peptide designation, wherein the left end indicates the N-terminus (amino terminus) and the right end indicates the C-terminus (carboxyl terminus). In the peptide complex contained as an active ingredient in the vaccine composition of the present invention, the C-terminus may be any of a carboxyl group (-COOH), carboxylate (-COO$^-$), amide (-CONH$_2$) and ester (-COOR).

Here, as R in the ester, a $C_{1-6}$ alkyl group, for example, methyl, ethyl, n-propyl, isopropyl and n-butyl, a $C_{3-8}$ cycloalkyl group, for example, cyclopentyl and cyclohexyl, a $C_{6-12}$ aryl group, for example, phenyl and $\alpha$-naphthyl, a phenyl-$C_{1-2}$ alkyl group, for example, benzyl and phenethyl, a $C_{7-14}$ aralkyl group, for example, an $\alpha$-naphthyl-$C_{1-2}$ alkyl group such as $\alpha$-naphthylmethyl, a pivaloyloxymethyl group; and the like can be used.

When the peptide complex has a carboxyl group (or carboxylate) at a position other than the C-terminus, a protein wherein the carboxyl group is amidated or esterified is also included in the peptide complex of the present invention. In this case, as the ester, for example, the above-described ester at the C terminus and the like are used.

Furthermore, the peptide complex also includes a peptide complex wherein the amino group of the N-terminal amino acid residue is protected by a protecting group (e.g., $C_{1-6}$ acyl groups such as $C_{1-6}$ alkanoyls such as formyl group and acetyl group, and the like); a peptide complex wherein an amino group of the N-terminal amino acid residue is acetylated; a peptide complex wherein a substituent (e.g., -OH, -SH, amino group, imidazole group, indole group, guanidino group and the like) on a side chain of an amino acid in the molecule is protected by an appropriate protecting group (e.g., $C_{1-6}$ acyl groups such as $C_{1-6}$ alkanoyl groups such as formyl group and acetyl group, and the like), and the like. In one embodiment, in the peptide complex, an amino group of the N-terminal amino acid residue is acetylated and/or a C-terminal carboxyl group is amidated.

The peptide complex to be contained as the active ingredient in the vaccine composition of the present invention contains a B cell receptor antigen peptide in a part thereof. As used herein, the B cell receptor is a receptor expressed on the surface of B cells. Stimulated by an antigen peptide, the B cells proliferate and secrete the B cell receptor as an antibody against antigen peptide.

The B cell receptor antigen peptide in the vaccine composition of the present invention may be a peptide containing an amino acid sequence represented by the formula (I): X1-X2-X3-X4-X5-X6-X7-X8 or a peptide consisting of the 8 amino acids, wherein

X1 is S (serine), A (alanine), G (glycine), T (threonine), K (lysine), or R (arginine),
X2 is P (proline), A (alanine), G (glycine), S (serine), T (threonine), K (lysine), or R (arginine),
X3 is S (serine), A (alanine), G (glycine), T (threonine), K (lysine), or R (arginine),
X4 is Q (glutamine), A (alanine), G (glycine), T (threonine), or N (asparagine),
X5 is P (proline), A (alanine), G (glycine), S (serine), T (threonine), Q (glutamine), or N (asparagine),
X6 is W (tryptophan), A (alanine), Y(tyrosine), or F (phenylalanine),
X7 is Q (glutamine), A (alanine), G (glycine), T (threonine), or N (asparagine), and
X8 is R (arginine), A (alanine), G (glycine), or K (lysine).

In one embodiment, X6 in the formula (I) may be W.
In one embodiment, X7 in the formula (I) may be Q.
In one embodiment, X8 in the formula (I) may be R.
In one embodiment, in the formula (I),

X1 may be S or A,
X2 may be P, A, S, or T,
X3 may be S or A,
X4 may be Q or A,
X5 may be P, A, S, T, or Q,
X6 may be W or A,
X7 may be Q or A, and
X8 may be R or A.

In one embodiment, in the formula (I),

X1 may be S or A,
X2 may be P, A, S, or T,
X3 may be S or A,
X4 may be Q or A,
X5 may be P, A, S, T, or Q,
X6 may be W or A,
X7 may be Q or A, and
X8 may be R.

In one embodiment, in the formula (I),

X1 may be S or A,
X2 may be P, A, S, or T,
X3 may be S,
X4 may be Q or A,
X5 may be P, A, S, T, or Q,
X6 may be W or A,
X7 may be Q or A, and
X8 may be R.

In one embodiment, in the formula (I),

X1 may be S or A,
X2 may be P, A, S, or T,
X3 may be S or A,
X4 may be Q,
X5 may be P, A, S, T, or Q,
X6 may be W or A,
X7 may be Q or A, and
X8 may be R.

In one embodiment, in the formula (I),

X1 may be S or A,
X2 may be P, A, S, or T,
X3 may be S or A,
X4 may be Q or A,
X5 may be P, A, S, T, or Q(provided that when X2 is S, X5 is P, A, or Q),
X6 may be W or A,
X7 may be Q or A, and
X8 may be R.

In one embodiment, in the formula (I),

X1 may be S,
X2 may be P, S, or T,
X3 may be S,
X4 may be Q,
X5 may be P, S, T, or Q(provided that when X2 is S, X5 is P or Q),
X6 may be W or A,
X7 may be Q or A, and
X8 may be R.

In one embodiment, in the formula (I),

X1 may be S,
X2 may be P, A, S, or T,
X3 may be S,
X4 may be Q or A,
X5 may be P,
X6 may be W or A,
X7 may be Q or A, and
X8 may be R.

In one embodiment, the 8 amino acids represented by the formula (I) may be any of the amino acid sequences shown in SEQ ID NOs: 2, 6 - 14 and 17 - 26.
A part of the complex contained in the vaccine composition of the present invention is a T cell receptor antigen peptide. The T cell receptor antigen peptide is not particularly limited as long as it is an antigen peptide that forms a complex with MHC class II, is recognized by CD3/TCR complex and CD4, and transmits signals into CD3-positive cells. MHC class II includes, for example, HLA-DR, HLA-DQ, and HLA-DP in the case of human, and H-2A and H-2B in the case of mouse, each of which is a dimer composed of α chain and β chain (e.g., HLA-DRA as α chain and HLA-DRB1 as β chain for HLA-DR), preferably HLA-DR, HLA-DQ, or HLA-DP.
In the vaccine composition of the present invention, the T cell receptor antigen peptide is not particularly limited as long as it contains an epitope sequence that can be presented on MHC class II molecules of antigen-presenting cells to activate helper T cells. For example, AJP001 peptide (ELKLIFLHRLKRLRKRLKRK (SEQ ID NO: 38), see WO2016/047763 for the detail) developed by the present inventors, UBITh peptide (UBITh (registered trade mark) 1: ISITEIKGVIVHRIETILF (SEQ ID NO: 39), UBITh (registered trade mark) 2: KKKIITITRIITIITTID (SEQ ID NO: 40), UBITh (registered trade mark) 3: ISISEIKGVIVHKIETILF (SEQ ID NO: 41), ISITEIRTVIVTRIETILF (SEQ ID NO: 42), see US patent no. 9,102,752 for the detail), Tetanous toxisoid peptide (TTaa830-843 peptide: QYIKANSKFIGITE (SEQ ID NO: 43)), and the like can be used. AJP001 induces the secretion of IL-β1 and IL-18 through activation of NLRP3 inflammasome, and can also activate the innate immune system by inducing the production of TNF-α and IL-6 through NF-κB pathway. Thus, it is particularly preferred as a T cell receptor antigen peptide in the present invention.
In the vaccine composition of the present invention, a T cell receptor antigen peptide and a B cell receptor antigen peptide are bonded to form a complex. The bond may be a linkage between the terminus of one peptide chain and the terminus of the other peptide chain, or a linkage between the amino acid side chain of one peptide and the terminus of the other peptide chain, or a linkage between the both amino acid side chains, preferably a linkage between the terminus of one peptide chain and the terminus of the other peptide chain, more preferably a linkage between the C-terminus of one peptide chain and the N-terminus of the other peptide chain, further preferably a linkage between the C-terminus of T cell receptor antigen peptide and the N-terminus of B cell receptor antigen peptide. When the terminus of one peptide chain and the terminus of the other peptide chain are linked, the terminal amino acids of the both may be directly linked by a peptide bond or via a linker (also to be referred to as "spacer" in the present specification). The linker is not particularly limited as long as it can connect a T cell receptor antigen peptide and a B cell receptor antigen peptide, can be taken up into an antigen presenting cell, can liberate the helper T cell epitope in the T cell receptor antigen peptide, and can present it on MHC class II molecules. For example, amino acids other than α-amino acid, such as ε-aminocaproic acid, β-aminoalanine, γ-aminobutyric acid, 7-aminoheptanoic acid, 12-aminolauric acid, p-aminobenzoic acid, and the like, can be used. In addition, L-amino acid (e.g., glutamic acid, cysteine, lysine) present in natural proteins and D-amino acids thereof can also be used. In one preferred embodiment, the amino acid linker is ε-aminocaproic acid. Alternatively, a peptide linker consisting of any 2 to 15 amino acids can also be used. Examples thereof include, but are not limited to, a G linker, which is a peptide linker composed of glycine (Gly) or methylated glycine (MeG), a GS linker, which is a peptide linker composed of Gly or MeG and Ser, and the like. In another embodiment, a PEG linker containing polyethylene glycol (PEG) or a derivative of polyethylene glycol can also be used. A PEG linker further containing one or more selected from glycine (Gly), serine (Ser), glutamic acid (Glu), arginine (Arg), and lysine (Lys) can also be used.

In one embodiment, a complex in which a B cell receptor antigen peptide and a T cell receptor antigen peptide are linked may further contain additional amino acids. Addition of amino acids is acceptable as long as the desired effect of the vaccine composition of the present invention is obtained. While the amino acid sequence to be added is not particularly limited, for example, a tag that facilitates detection, purification and the like of the complex can be mentioned. Examples of the tag include Flag tag, histidine tag, c-Myc tag, HA tag, AU1 tag, GST tag, MBP tag, fluorescence protein tag (e.g., GFP, YFP, RFP, CFP, BFP etc.), immunoglobulin Fc tag, and the like. The position at which the amino acid sequence is added is not particularly limited, and is preferably the N-terminus and/or C-terminus of the complex.

A complex in the vaccine composition of the present invention can be produced by a solid phase synthesis process (Fmoc method and Boc method) or a liquid phase synthesis process, according to a known general protocol of peptide synthesis. When it is a complex in which a B cell receptor antigen peptide and a T cell receptor antigen peptide are linked directly or via an amino acid or peptide linker, the whole complex can be synthesized at once. Alternatively, a B cell receptor antigen peptide and a T cell receptor antigen peptide may be separately synthesized and thereafter the two peptides may be linked directly or via a linker.

In one embodiment, the peptide complex in the vaccine composition of the present invention may be conjugated with a carrier protein in order to increase the immunogenicity thereof. A carrier protein is generally a substance that binds to a molecule having no immunogenicity (hapten) due to its small molecular weight and imparts the immunogenicity, and is known in the technical field. Preferable examples of the carrier protein include bovine serum albumin (BSA), rabbit serum albumin (RSA), ovalbumin (OVA), keyhole-limpet hemocyanin (KLH), thyroglobulin (TG), diphtheria toxin made non-toxic by replacing some of its amino acids (CRM197), immunoglobulin, and the like. A carrier protein can be conjugated to the N-terminus or C-terminus of the complex in the vaccine composition of the present invention. Methods for conjugation include introducing a cysteine residue into the antigen peptide of the present invention, and binding the peptide to the amino group of the carrier protein via an SH group which is the side chain of cysteine (MBS method). In addition, amino groups such as ε amino group, α amino group and the like of the lysine residue of a protein can also be bound to each other (glutaraldehyde method) to perform conjugation.

In a preferred embodiment, the complex in the vaccine composition of the present invention is not conjugated with a carrier protein. When the complex is taken up into an antigen presenting cell, the helper T cell epitope in the T cell receptor antigen peptide is liberated and presented on MHC class II molecules, recognized by a T cell receptor on the helper T cell, and activates the helper T cell. The activated helper T cell recognizes B cells that present the helper T cell epitopes on the MHC class II molecule in the same way, secretes cytokines such as IFN-γ and the like, activates the B cells and can strongly promote the production of antibodies against the B cell receptor antigen peptide (IL-23 p19). Therefore, high antibody production can be induced without using a carrier protein. When a carrier protein is used in combination with a peptide vaccine, antibodies specific to the carrier protein may be induced to cause undesired side effects. Furthermore, peptide vaccines conjugated with carrier proteins are not preferred because the production cost increases.

In one embodiment, the vaccine composition of the present invention may further contain a pharmaceutically acceptable adjuvant compatible with the active ingredient. Adjuvant is generally a substance that non-specifically potentiates an immune response of the host, and a number of adjuvants are known in the technical field. The adjuvant used for the vaccine composition of the present invention is not particularly limited as long as it can non-specifically potentiate immune responses. For example, aluminum salt (Alum), alum, CpG oligo deoxynucleotide, dsRNA, montanide, squalane, saponin, and the like can be mentioned.

In a preferred embodiment, the vaccine composition of the present invention is not used in combination with an adjuvant. For example, when a peptide capable of activating the innate immune system such as AJP001 and the like is used as a T cell receptor antigen peptide, since the vaccine composition of the present invention alone can sufficiently induce antibody production, it is not necessary to use an adjuvant in combination. The use of adjuvants may induce unexpected side reactions, and not using them reduces safety risks.

The vaccine composition of the present invention can be provided as a pharmaceutical composition containing a pharmaceutically acceptable carrier in addition to a complex of a T cell receptor antigen peptide and a B cell receptor antigen peptide.

The pharmaceutically acceptable carrier may be selected as appropriate according to the dosage form. Examples thereof include, but are not limited to, excipients such as sucrose, starch and the like, binders such as cellulose, methylcellulose and the like, disintegrants such as starch, carboxymethylcellulose and the like, lubricants such as magnesium stearate, and the like, aromatics such as citric acid, menthol and the like, preservatives such as sodium benzoate, sodium bisulfite and the like, stabilizers such as sodium citrate and the like, suspending agents such as methylcellulose, polyvinylpyrrolidone and the like, dispersing agents such as surfactant and the like, diluents such as water, saline and the like, base-wax and the like.

The vaccine composition of the present invention can be administered orally or parenterally to a mammal. Since a complex of a T cell receptor antigen peptide and a B cell receptor antigen peptide can be decomposed in the stomach, parenteral administration is preferable. A preparation preferable for oral administration includes liquid, capsule, sachet, tablet, suspension, emulsion and the like. A preparation preferable for parenteral administration (e.g., subcutaneous injection, muscular injection, topical injection, intraperitoneal administration and the like) includes aqueous and non-aqueous isotonic, aseptic injection liquid, which optionally contains antioxidant, buffer, bacteriostatic agent, isotonicity agent and the like. Also, an aqueous and non-aqueous aseptic suspending agent can be mentioned, and the agent optionally contains suspending agent, solubilizer, thickener, stabilizer, preservative and the like. Such preparation can be sealed in a unit dose or plural dose container such as ampoule or vial. In addition, the active ingredient and pharmaceutically acceptable carriers may be freeze-dried, and preserved in a form only requiring dissolution or suspending in a suitable aseptic vehicle immediately before use.

The content of the active ingredient (i.e., peptide complex) in a vaccine composition is, but not limited to, generally about 0.001 - 100 wt%, preferably about 0.05 - 99 wt%, further preferably 0.1 - 90 wt%, of the whole composition.

The subject of administration of the vaccine composition of the present invention is not particularly limited as long as the subject may be affected with a disease associated with abnormal enhancement of signal transduction involving IL-23 (hereinafter sometimes referred to as "IL-23-associated disease"). Examples of the mammal include rodents such as mouse and the like, pets such as dog and the like, domestic animals such as swine, horse, bovine and the like, primates such as human, monkey, orangutan, chimpanzee and the like, and the like, and human is particularly preferred.

The dose of the vaccine composition of the present invention varies depending on the recipient of administration, administration method, administration form and the like. Generally, 1 μg - 300,000 μg, preferably 20 μg - 30,000 μg, of a complex of a T cell receptor antigen peptide and a B cell receptor antigen peptide as the active ingredient is administered per dose to an adult 2 or 3 times for generally 4 weeks to 12 weeks. When the antibody titer falls, additional administration can be performed once each time.

Diseases that may be treated or prevented by the vaccine compositions of the invention are the aforementioned IL-23-associated diseases. Examples of the disease include, but are not limited to, psoriasis, psoriatic arthritis, rheumatoid arthritis, systemic lupus erythematosus, diabetes (preferably type I diabetes), atherosclerosis, inflammatory bowel disease (IBD)/Crohn's disease, multiple sclerosis, Behcet's disease, ankylopoietic spondylarthritis, Vogt-Koyanagi-Harada disease, chronic granulomatous disease, hidradenitis suppurativa, Anti-Neutrophil Cytoplasmic Antibody (ANCA) Associated Vasculitis, neurodegenerative disease (preferably Alzheimer's disease or multiple sclerosis), atopic dermatitis, graft-versus-host disease or cancer (preferably, esophageal cancer (oesophagal carcinoma), colorectal cancer, lung adenocarcinoma, small cell cancer, and flat epithelial cancer in mouth cavity).

In one embodiment, the vaccine composition of the present invention may be used to treat or prevent psoriasis, psoriatic arthritis, neurodegenerative disease (particularly Alzheimer's disease), diabetes (particularly type I diabetes), atherosclerosis, or IBD. In a particularly preferred embodiment, the vaccine composition of the present invention may be used to treat or prevent psoriasis, psoriatic arthritis, or IBD.

In one embodiment, the vaccine composition of the present invention may be used in combination with existing therapeutic agents for IL-23-associated diseases. For example, in the treatment of psoriasis, both the patients' QOL and treatment cost can be achieved by starting treatment with an immediately highly effective antibody drug during the active stage of the disease and starting administration of the vaccine composition of the present invention during the remission stage.

Note that the "treatment" of a disease in the present description can include not only cure of the disease but also remission of the disease and improvement of the severity of the disease.

Further, the "prevention" of a disease in the present description includes, in addition to prevention of the onset of the disease, delaying of the onset of the disease. In addition, the "prevention" of a disease in the present description can also include prevention of the recurrence of the disease after treatment, or delaying of the recurrence of the disease after treatment.

Further, the term "vaccine composition" in the present description can also be rephrased as "pharmaceutical composition" or "agent".

## 2. Method for treating or preventing IL-23-associated disease

The present invention also provides a method for treating or preventing an IL-23-associated disease, including administering the vaccine composition of the present invention to a subject suffering from an IL-23-associated disease (hereinafter sometimes referred to as "the method of the present invention").

In the method of the present invention, the subject of treatment or prevention, administration conditions for the vaccine composition of the present invention, specific examples of IL-23-associated disease, and the like are the same as those described in "1. the vaccine composition of the present invention".

## 3. B cell receptor antigen peptide

The present invention also provides a B cell receptor antigen peptide itself that constitutes a part of the peptide complex contained in the vaccine composition of the present invention (hereinafter sometimes referred to as "the B cell receptor antigen peptide of the present invention").

The B cell receptor antigen peptide of the present invention may be a peptide containing an amino acid sequence represented by the formula (I): X1-X2-X3-X4-X5-X6-X7-X8 or a peptide consisting of the 8 amino acids, wherein

X1 is S (serine), A (alanine), G (glycine), T (threonine), K (lysine), or R (arginine),
X2 is P (proline), A (alanine), G (glycine), S (serine), T (threonine), K (lysine), or R (arginine),
X3 is S (serine), A (alanine), G (glycine), T (threonine), K (lysine), or R (arginine),
X4 is Q (glutamine), A (alanine), G (glycine), T (threonine), or N (asparagine),
X5 is P (proline), A (alanine), G (glycine), S (serine), T (threonine), Q (glutamine), or N (asparagine),
X6 is W (tryptophan), A (alanine), Y(tyrosine), or F (phenylalanine),
X7 is Q (glutamine), A (alanine), G (glycine), T (threonine), or N (asparagine), and
X8 is R (arginine), A (alanine), G (glycine), or K (lysine).

In one embodiment, X6 in the formula (I) may be W.
In one embodiment, X7 in the formula (I) may be Q.
In one embodiment, X8 in the formula (I) may be R.
In one embodiment, in the formula (I),

X1 may be S or A,
X2 may be P, A, S, or T,
X3 may be S or A,
X4 may be Q or A,
X5 may be P, A, S, T, or Q,
X6 may be W or A,
X7 may be Q or A, and
X8 may be R or A.

In one embodiment, in the formula (I),

X1 may be S or A,
X2 may be P, A, S, or T,
X3 may be S or A,
X4 may be Q or A,
X5 may be P, A, S, T, or Q,
X6 may be W or A,
X7 may be Q or A, and
X8 may be R.

In one embodiment, in the formula (I),

X1 may be S or A,
X2 may be P, A, S, or T,
X3 may be S,
X4 may be Q or A,
X5 may be P, A, S, T, or Q,

X6 may be W or A,
X7 may be Q or A, and
X8 may be R.

In one embodiment, in the formula (I),

X1 may be S or A,
X2 may be P, A, S, or T,
X3 may be S or A,
X4 may be Q,
X5 may be P, A, S, T, or Q,
X6 may be W or A,
X7 may be Q or A, and
X8 may be R.

In one embodiment, in the formula (I),

X1 may be S or A,
X2 may be P, A, S, or T,
X3 may be S or A,
X4 may be Q or A,
X5 may be P, A, S, T, or Q (provided that when X2 is S, X5 is P, A, or Q),
X6 may be W or A,
X7 may be Q or A, and
X8 may be R.

In one embodiment, in the formula (I),

X1 may be S,
X2 may be P, S, or T,
X3 may be S,
X4 may be Q,
X5 may be P, S, T, or Q (provided that when X2 is S, X5 is P or Q),
X6 may be W or A,
X7 may be Q or A, and
X8 may be R.

In one embodiment, in the formula (I),

X1 may be S,
X2 may be P, A, S, or T,
X3 may be S,
X4 may be Q or A,
X5 may be P,
X6 may be W or A,
X7 may be Q or A, and
X8 may be R.

In one embodiment, the 8 amino acids represented by the formula (I) may be any of the amino acid sequences shown in SEQ ID NOs: 2 and 6 - 14 and 17 - 26.

The B cell receptor antigen peptide of the present invention can be used alone as a peptide vaccine to induce an anti-IL-23 antibody. In a more preferred embodiment, it can be used as a peptide vaccine with higher immunogenicity by combining with a T cell antigen receptor peptide. In a particularly preferred embodiment, the B cell antigen receptor peptide of the present invention can be used as a peptide vaccine that can efficiently induce anti-IL-23 antibody by using in a conjugated form with AJP001.

The present invention is more specifically described in the following Examples, but the present invention is by no means limited by these examples.

14

[Example]

<u>Synthesis of peptide (Fmoc method)</u>

A protected peptide resin was synthesized by the Fmoc method according to the method described in 5th Edition, Jikken Kagaku Kouza 16, Synthesis of Organic Compounds IV and the like and using a fully automatic solid phase synthesizer. To the obtained protected peptide resin were added trifluoroacetic acid (TFA) and Scavenger (mixture of thioanisole, 2,2'-(ethylenedioxy)di-ethanethiol, m-cresol, triisopropyl silane, water, and the like), and a crude peptide was obtained by excising from the resin, followed by deprotection. The crude peptide was purified using a reverse phase HPLC column with gradient elution using a 0.1% TFA-$H_2O$/$CH_3CN$ system. Fractions containing the target product were collected and lyophilized to obtain the peptide of interest.

<u>HPLC analysis method for peptide</u>

The purity of the synthesized peptide was measured using an HPLC device under the following analysis conditions.

　　HPLC model: Shimadzu Corporation LCLC-20ADXR
　　measurement wavelength: 220 nm
　　flow: 0.31 mL per minute
　　column: Inertsil ODS-3, 2.1m×250mm, 5 micron
　　column temperature: room temperature
　　mobile phase A: 0.1% trifluoroacetic acid aqueous solution mobile phase B: acetonitrile
　　gradient conditions: linear gradient of mobile phase B concentration from 5% to 80% in 30 min (5 → 80% buffer B in 30 min)

<u>Mass spectrometry of peptide</u>

The mass of the synthesized peptide was measured by MALDI-TOF-MS under the following analysis conditions.

　　MALDI-TOF-MS model: Bruker autoflex speed matrix: 2,5-Dihydroxybenzoic acid
　　dissolve solution: mixed solution of 0.1% trifluoroacetic acid aqueous solution and acetonitrile

[Example 1] Antibody production evaluation test using AJP001 conjugated peptide (mouse IL-23)

The epitope peptides derived from mouse IL-23 shown in Table 1 (SEQ ID NOs: 1 to 5, Fig. 1(a)) were selected as B cell antigens, and using T cell antigen AJP001 (Table 2, SEQ ID NO: 38) and ε-aminocaproic acid (sometimes referred to as "Ahx") as a spacer, conjugated complex (AJP001 conjugated peptide) was produced (production was outsourced to PEPTIDE INSTITUTE, INC.). In the present specification, the AJP001 conjugated peptide whose B cell epitope has the amino acid sequence shown in SEQ ID NO: "X" is to be referred to as "AJP001 conjugated peptide (SEQ ID NO: X)" and the like.

[Table 1]

| SEQ ID NO | species | amino acid position | B cell epitope sequence | mass* (MW) | purity* (%) |
|---|---|---|---|---|---|
| 1 | mouse | 151-158 | LSSSQQWQ | 3744.2 | 98.1 |
| 2 | | 152-159 | SSSQQWQR | 3787.4 | 98.6 |
| 3 | | 153-160 | SSQQWQRP | 3797.3 | 99.1 |
| 4 | | 154-161 | SQQWQRPL | 3823.3 | 97.7 |
| 5 | | 156-163 | QWQRPLLR | 3877.6 | 98.9 |
| 6 | human | 151-158 | SPSQPWQR | 3765.2 | 98.2 |

(continued)

| SEQ ID NO | species | amino acid position | B cell epitope sequence | mass* (MW) | purity* (%) |
|---|---|---|---|---|---|
| 7 | artificial sequence | 151-158 | APSQPWQR | 3749.19 | 98.74 |
| 8 | | 151-158 | SASQPWQR | 3739.08 | 97 |
| 9 | | 151-158 | SPAQPWQR | 3749.43 | 98.45 |
| 10 | | 151-158 | SPSAPWQR | 3708.04 | 96.32 |
| 11 | | 151-158 | SPSQAWQR | 3739.56 | 98.13 |
| 12 | | 151-158 | SPSQPAQR | 3650.41 | 97.97 |
| 13 | | 151-158 | SPSQPWAR | 3708.42 | 98.53 |
| 14 | | 151-158 | SPSQPWQA | 3680.35 | 98.17 |
| 15 | | - | PWQRPWQR | 3933.31 | 97.55 |
| 16 | | - | QWQRPWQR | 3964.46 | 98.37 |
| 17 | | 151-158 | SSSQPWQR | 3755.13 | 98.83 |
| 18 | | 151-158 | STSQPWQR | 3769.23 | 97.25 |
| 19 | | 151-158 | SPSQSWQR | 3755.18 | 98.54 |
| 20 | | 151-158 | SPSQTWQR | 3769.59 | 97.86 |
| 21 | | 151-158 | SSSQSWQR | 3745.06 | 98.62 |
| 22 | | 151-158 | SSSQTWQR | 3759.55 | 98 |
| 23 | | 151-158 | STSQSWQR | 3759.59 | 98.09 |
| 24 | | 151-158 | STSQTWQR | 3773.56 | 98.89 |
| 25 | | 151-158 | SPSQQWQR | 3796.53 | 99.04 |
| 26 | | 151-158 | STSQQWQR | 3800.01 | 97.84 |
| 27 | | 151-158 | KPSQPWQR | 3806.56 | 96.15 |
| 28 | | 151-158 | SKSQPWQR | 3796.44 | 96.12 |
| 29 | | 151-158 | SPKQPWQR | 3806.2 | 96.12 |
| 30 | | 151-158 | KKSQPWQR | 3837.3 | 96.6 |
| 31 | human | 152-158 | PSQPWQR | 3678.26 | 95.64 |
| 32 | | 153-158 | SQPWQR | 3581.18 | 96.18 |
| 33 | | 154-158 | QPWQR | 3493.93 | 95.75 |
| 34 | | 152-159 | PSQPWQRK | 3806.11 | 95.8 |
| 35 | | 153-159 | SQPWQRK | 3709.1 | 95.02 |
| 36 | artificial sequence | - | QPWQRSQPWQR | 4276.3 | 91.61 |
| 37 | | - | QPWQRGGGGSQPWQR | 4507.74 | 91. 6 |
| * property value of AJP001-Ahx-B cell epitope peptide conjugate obtained by chemical synthesis | | | | | |

[Table 2]

| SEQ ID NO | amino acid sequence |
|---|---|
| 38 | ELKLIFLHRLKRLKRLKRK |

AJP001 conjugated peptide having one of the B cell epitope sequences of SEQ ID NOs: 1 - 5 was dissolved in physiological saline and intradermally administered twice to BALB/c mice (female, 6-week-old, N=5 - 6) at 0.5 mg/body at 2-week intervals. Blood was collected before administration and 5 weeks after the initial administration, and the antibody titer against each AJP001 conjugated peptide and the binding ability to recombinant mouse IL-23 protein (rmIL-23) were measured by the ELISA method. Specifically, a 96-well plate immobilized with an epitope peptide dissolved at 10 $\mu$g/mL in carbonate buffer was blocked with 5% skim milk/PBS, and then serum serially diluted with 5% skim milk/PBS was added, and the mixture was left standing at 4°C overnight. After washing the wells with PBS-T, HRP-labeled anti-mouse IgG antibody (GE Healthcare) diluted with 5% skim milk/PBS was added and the mixture was shaken at room temperature for 3 hr. After washing the wells with PBS-T, TMB solution (SIGMA) was added, the wells were allowed to stand for 30 min in the dark, the reaction was stopped by adding 0.5M $H_2SO_4$, and the absorbance at 450 nm was measured using a plate reader. The serum dilution rate that gave 1/2 (OD=1.75) of the maximum absorbance was defined as the antibody titer, and the geometric mean titer:GMT was calculated from the individual values. Binding to IL-23 protein was measured by the ELISA method in the same manner as the antibody titer, using rmIL-23 (Antibodies-online GmbH) as the solid-phased antigen and goat serum for blocking. The antibody titer and binding activity to rmIL-23 of each AJP001 conjugated peptide are shown in Fig. 1 (b) and (c). As a result, a significant increase in antibody titer and binding to rmIL-23 were confirmed in the antiserum of mice administered with AJP001 conjugated peptide having the B cell epitope sequence of SEQ ID NO: 2 (AJP001 conjugated peptide (SEQ ID NO: 2)).

[Example 2] Mouse immunogenicity evaluation test using AJP001 conjugated peptide (SEQ ID NO: 2)

AJP001 conjugated peptide (SEQ ID NO: 2) was dissolved in physiological saline and intradermally administered to BALB/c mice (female, 6-week-old, N=6) at 1 mg/body 3 times at two-week intervals, and blood was collected every 2 weeks until 8 weeks. The antibody titer (GMT) of all the collected sera and the binding ability to rmIL-23 of the serum at the time point of 6 weeks were measured by the ELISA method (same method as in Example 1). The antibody titer and the binding of the induced antibody to rmIL-23 at each time point are shown in Fig. 2 (a) and (b), respectively.

As shown in Fig. 2 (a) and (b), in the antiserum of mice administered with the AJP001 conjugated peptide (SEQ ID NO: 2), the antibody titer increased from the 2nd week and persisted until the 8th week. Furthermore, it was confirmed that the antiserum at the time point of 6 weeks later contained antibodies that bind to rmIL-23.

In addition, ELISPOT assay was performed to evaluate T cell activation. AJP001 conjugated peptide (SEQ ID NO: 2) or physiological saline was administered three times at two-week intervals to BALB/c mice (female, 6-week-old, N=3 - 4), and the spleen was collected 7 weeks after the first administration. The collected spleen was triturated using a syringe barrel and a cell strainer. This was washed with and suspended in HBSS (-), erythrocytes were removed using ACK Buffer (Thermo fisher scientific), and splenocytes were isolated. Using Mouse IFN-$\gamma$ ELISpot assay kit and Mouse IL-4 ELISpot assay kit (R&D systems), the cells were prepared to 2.5 $\times$ 10^6 cells/mL with 10 %FBS/1% penicillin/streptomycin/4 ng/mL rmIL-2-containing RPMI1640 medium (culture medium), and seeded at 100 $\mu$L/well on the 96-well plate of which each capture antibody was immobilized. Splenocytes were stimulated by adding (1) medium alone, (2) 10 $\mu$g/mL T cell antigen AJP001 (SEQ ID NO: 38) alone, (3) 10 ug/mL AJP001 conjugated peptide (SEQ ID NO: 2), or (4) 100 ng/mL PMA/ionomycin (PMA/IM, Sigma) to each well. Stimulation was performed for 2 days in a $CO_2$ incubator. Then, a detection antibody was added and, after washing, a colorimetric reaction was performed. The stained cells were measured with a microscope. The number of IFN-$\gamma$ and IL-4 positive cells was calculated as the number of cells per 10^6 cell number (SFC/10^6 cells). The results are shown in Figs. 2 (c) and (d). In Fig. 2, "*" means significant (P<0.05).

As shown in Figs. 2 (c) and (d), in the splenocytes of the mice administered with the AJP001 conjugated peptide (SEQ ID NO: 2), an increase in the number of IFN-$\gamma$ and IL-4 positive cells was confirmed by stimulation with (2) 10 $\mu$g/mL T cell antigen AJP001 (SEQ ID NO: 38) alone, and (3) 10 ug/mL AJP001 conjugated peptide (SEQ ID NO: 2). As a result, AJP001 conjugated peptide-specific activation of T cells was confirmed.

[Example 3] Efficacy evaluation test using AJP001 conjugated peptide (SEQ ID NO: 2) in psoriasis model

AJP001 conjugated peptide (SEQ ID NO: 2) was dissolved in physiological saline and intradermally administered to hairless rats (female, 6-week-old, N=6) at 1 mg/body 3 times at two-week intervals. From 6 weeks after the initial administration, 2% imiquimod (IMQ) was intermittently applied to the auricle 4 times in total at 1-day intervals to create a psoriasis model. A 2% IMQ application group was provided as a control group.

The drug efficacy was evaluated by measuring the thickness of the auricle with a thickness gauge (Mitutoyo Corporation) from the first day of IMQ application to the eighth day. Furthermore, on the 8th day, histopathological evaluation of the auricular tissue was performed. Specifically, the collected auricle was fixed with 10% neutral formalin, and then a specimen was prepared according to a conventional method and stained with HE. HE-stained specimens were observed under a microscope, and inflammatory cell infiltration, edema, and thickness were scored in 4 stages (0: normal, 1: mild, 2: intermediate, 3: severe) and the total score was calculated. Furthermore, the antibody titer (GMT) of the serum at the time point of 6 weeks was measured by the ELISA method (same method as in Example 1. Anti-Rat IgG (GE Healthcare) was used as the secondary antibody). The results of the antibody titer, auricular thickness, and histological test of the AJP001 conjugated peptide (SEQ ID NO: 2) are shown in Fig. 3. In Fig. 3, "*" means that it is significant (P<0.05) compared with "2% IMQ". "#" means that it is significant (P<0.05) compared with "Day 0" in (b) and "Normal (normal group)" in (d).

As shown in Fig. 3, an increase in the antibody titer was confirmed in the antiserum of the hairless rats administered with AJP001 conjugated peptide (SEQ ID NO: 2) (Fig. 3 (a)). In the group administered with AJP001 conjugated peptide (SEQ ID NO: 2), a suppressive action on the auricle inflammatory responses (increase in auricular thickness and inflammatory changes of tissue) observed in the medium control group was confirmed (Figs. 3 (b), (c) and (d)).

[Example 4] Efficacy evaluation test using AJP001 conjugated peptide (SEQ ID NO: 2) in IL-23-induced mouse auricle inflammation model

AJP001 conjugated peptide (SEQ ID NO: 2) was dissolved in physiological saline and intradermally administered to BALB/c mice (female, 6-week-old, N=8) at 1 mg/body 3 times at two-week intervals. At the time point of 6 weeks after the initial administration, rmIL-23 was administered intradermally to the ear to induce inflammation. With the first administration as Day 0, rmIL-23 was administered 4 times in total on Days 2, 4, and 7. As a control group, an rmIL-23 group without administration of peptide was provided. Auricular thickness was measured using a thickness gauge on each day of administration of rmIL-23 and on Day 9. In order to measure the mRNA expression of IL-17A, IL-22, IL-1$\beta$, LCN-2, and CXCL-2 in the auricle, the auricle was collected on the third day of rmIL-23 administration under the same conditions. For mRNA evaluation, a normal group without administration of rmIL-23 was set. The collected auricles were frozen and crushed under liquid nitrogen, then total RNA was extracted using RNeasy Fibrous Tissue Kit (Qiagen), and then cDNA of the extracted RNA was prepared using High-Capacity cDNA Reverse Transcription Kit (Applied Biomedical Inc). Using TaqMan Gene Expression Assays (S18: Mm02601777_g1; CXCL-2: Mm00436450_m1; IL-17A: Mm00439618_m1; IL-22: Mm01226722_m1; IL-1$\beta$: Mm00434228_m1; LCN-2: Mm01324470_m1), Ct value was measured using Applied Biosystems 7900HT Fast Real Time PCR System. The mRNA expression level was calculated by the $\Delta\Delta$Ct method and evaluated as a relative value to the normal group. The results of efficacy evaluation of the AJP001 conjugated peptide (SEQ ID NO: 2) on auricular thickness and mRNA expression are shown in Fig. 4. In Fig. 4, "*" means that it is significant (P<0.05) compared with "rmIL-23". "#" means that it is significant (P<0.05) compared with "Day 0" in (a) and "Normal (normal group)" in (b).

As shown in Fig. 4 (a) and (b), a suppressive action on auricle inflammatory response (increase in auricular thickness and mRNA expression of IL-17A, IL-22, IL-1$\beta$, LCN-2, and CXCL-2 in auricle) observed in the rmIL-23 group was confirmed in the mice administered with AJP001 conjugated peptide (SEQ ID NO: 2).

Example 5] Antibody production and activity evaluation using AJP001 conjugated peptide (human IL-23)

The epitope candidate peptides derived from human IL-23 shown in Table 1 (SEQ ID NO: 6 - 37) were selected as B cell antigens, and using T cell antigen AJP001 (Table 2, SEQ ID NO: 38) and $\varepsilon$-aminocaproic acid as a spacer, conjugated complex (AJP001 conjugated peptide) was synthesized (synthesis was outsourced to Toray Research Center, Inc.).

AJP001 conjugated peptide (SEQ ID NOs: 6 - 37) was dissolved in physiological saline, mixed with an equal amount of 2% Alhydrogel (invivogen), and this was intradermally administered to BALB/c mice (female, 6-week-old, N=5 - 6) at 0.5 mg/body 3 times at two-week intervals. Blood was collected before administration and at the time point of 6 weeks after initial administration, and the antibody titer (GMT) to each epitope sequence and binding ability to recombinant human IL-23 protein (rhIL-23) (Peprotech) were measured by the ELISA method (same method as in Example 1). The binding to rhIL-23 was evaluated by a binding ratio to normal serum by calculating the ratio of the absorbance of a well added with normal mouse serum to the absorbance of a well added with antiserum derived from a mouse administered with each AJP001 conjugated peptide (SEQ ID NOs: 6 - 37). In addition, the neutralization activity of mouse antiserum was evaluated using an action on IL-17 production in rhIL-23 stimulated mouse splenocyte as an index. The spleen of normal BALB/c mouse (female, 5-week-old, N=3) was triturated using a syringe barrel and a cell strainer and washed with and suspended in HBSS (-). Erythrocytes were then removed using ACK Buffer (Thermo fisher scientific), and splenocytes were isolated. The splenocytes were prepared to $2.5 \times 10^6$ cells/mL with 10 %FBS/1% penicillin/streptomycin/4 ng/mL rmIL-2-containing RPMI1640 medium (culture medium), and seeded in a 96 well plate at 100 $\mu$L/well. The antiserum (final concentration 15-fold) of the mice administered with each AJP001 conjugated peptide and rhIL-23 (final concentration 10 ng/mL) were added to the culture medium, reacted at 37°C for 2 hr, added at 100 $\mu$L/well to a 96-well plate seeded with splenocytes, and cultured for 3 days in a 37°C/5% $CO_2$ incubator. An untreated well and a control well to which normal mouse serum and rhIL-23 were added were provided. IL-17A/F in the culture supernatant was measured using Mouse IL-17A/F ELISA kit (R&D systems), the proportion (%) of IL-17 to normal serum was calculated from the IL-17 concentrations of untreated well (A), control well (B), and well (C) to which antiserum of the mice administered with each AJP001 conjugated peptide was added and according to the following formula, and the neutralization activity was evaluated.

$$\text{Percentage of IL-17 to normal serum (\%)} = 100 \times (C-A/B-A)$$

The antibody titer, binding to rhIL-23, and neutralization activity of each AJP001 conjugated peptide are shown in Table 3.

[Table 3]

| SEQ ID NO | antibody titer (GMT) | binding to IL-23 (binding ratio to normal serum) | neutralization activity (percentage of IL-17 to normal serum (%)) |
|---|---|---|---|
| 6 | 12563 | 4.40 | 15.8 |
| 7 | 5417 | 1.40 | 4.2 |
| 8 | 3485 | 1.37 | 1.4 |
| 9 | 4683 | 1.01 | 4.0 |
| 10 | 19795 | 1.03 | 0.0 |
| 11 | 10441 | 1.55 | 8.1 |
| 12 | 48594 | 1.12 | 3.1 |
| 13 | 3215 | 1.11 | 4.8 |
| 14 | 3331 | 1.23 | 8.0 |
| 15 | 458 | 1.16 | 10 |
| 16 | 201 | 1.09 | 3.6 |
| 17 | 6096 | 1.26 | 1.6 |
| 18 | 8211 | 1.21 | 0.0 |
| 19 | 18311 | 1.33 | 2.4 |
| 20 | 36347 | 1.23 | 5.8 |
| 21 | 19793 | 1.07 | 6.9 |
| 22 | 2160 | 1.35 | 8.5 |
| 23 | 31720 | 1.33 | 9.4 |
| 24 | 126353 | 1.20 | 0.0 |
| 25 | 285265 | 1.66 | 2.1 |
| 26 | 64652 | 1.15 | 0.0 |
| 27 | 12615 | 1.07 | 72.3 |
| 28 | 4324 | 1.13 | 42.2 |
| 29 | 9056 | 1.11 | 63.9 |
| 30 | 6917 | 1.2 | 68.9 |
| 31 | 12417 | 0.99 | 63.4 |
| 32 | 5567 | 1.03 | 61.3 |
| 33 | 16458 | 1.00 | 72.6 |
| 34 | 362 | 0.97 | 32.5 |
| 35 | 92 | 1.30 | 32.5 |
| 36 | 8202 | 1.10 | 70.3 |

(continued)

| SEQ ID NO | antibody titer (GMT) | binding to IL-23 (binding ratio to normal serum) | neutralization activity (percentage of IL-17 to normal serum (%)) |
|---|---|---|---|
| 37 | 6459 | 1.05 | 63.9 |

As shown in Table 3, an increase in the antibody titer and binding to IL-23 were confirmed in the antiserum of the mice administered with respective AJP001 conjugated peptides. In addition, in the antiserum administered with each sequence, the inhibition of IL-17 production (neutralization activity) was confirmed.

[Example 6] Mouse immunogenicity evaluation test using AJP001 conjugated peptide (SEQ ID NO: 6)

AJP001 conjugated peptide (SEQ ID NO: 6) was dissolved in physiological saline and intradermally administered to BALB/c mice (female, 6-week-old, N=6) at 1 mg/body 3 times at two-week intervals, and blood was collected every 2 weeks until 8 weeks. The antibody titer (GMT) of all the collected sera and the binding ability to rhIL-23 of the serum at 6 weeks were measured by the ELISA method. The results of the antibody titer and binding of the AJP001 conjugated peptide (SEQ ID NO: 6) to rhIL-23 are shown in Figs. 5(a) and (b). In Fig. 5, "*" means P<0.05.
As shown in Figs. 5 (a) and (b), in the antiserum of mice administered with the AJP001 conjugated peptide (SEQ ID NO: 6), the antibody titer increased from the 2nd week and persisted until the 8th week. Furthermore, binding to rhIL-23 was confirmed in the antiserum 6 weeks later.
In addition, AJP001 conjugated peptide (SEQ ID NO: 6) or physiological saline was administered three times at two-week intervals to BALB/c mouse (female, 6-week-old, N=3 - 4), and the spleen was collected 7 weeks after the first administration. The collected spleen was subjected to ELISPOT assay using Mouse IFN-γ ELISpot assay kit and Mouse IL-4 ELISpot assay kit (R&D systems). The results are shown in Figs. 5 (c) and (d).
As shown in Figs. 5 (c) and (d), in the splenocytes derived from mice administered with the AJP001 conjugated peptide (SEQ ID NO: 6), an increase in the number of IFN-γ and IL-4 positive cells was confirmed by stimulation with 10 μg/mL T cell antigen AJP001 (SEQ ID NO: 38) and by stimulation with 10 μg/mL AJP001 conjugated peptide (SEQ ID NO: 6). As a result, AJP001 conjugated peptide-specific activation of T cells was confirmed.

[Example 7] Efficacy evaluation test using AJP001 conjugated peptide (SEQ ID NO: 6) in IL-23-induced mouse auricle inflammation model

AJP001 conjugated peptide (SEQ ID NO: 6) was dissolved in physiological saline and administered to BALB/c mice (female, 6-week-old, N=8) at 1 mg/body 3 times at two-week intervals. At the time point of 6 weeks after the initial administration, rhIL-23 was administered intradermally to the ear to induce inflammation. rhIL-23 was administered every day and 4 times in total. As a control group, an rhIL-23 group without administration of AJP001 conjugated peptide was provided. Auricular thickness was measured using a thickness gauge at the time points of 3 days and 4 days after rhIL-23 administration. In addition, the mRNA expression of IL-17A, IL-22, IL-1β, LCN-2, and CXCL-2 in the auricle was measured. The results of efficacy evaluation of the AJP001 conjugated peptide (SEQ ID NO: 6) on auricular thickness and mRNA expression are shown in Fig. 6. In Fig. 6, "*" means that it is significant (P<0.05) compared with "rhIL-23". "#" means that it is significant (P<0.05) compared with "Normal (normal group)".
As shown in Fig. 6, a suppressive action on auricle inflammatory response (increase in auricular thickness and mRNA expression of IL-17A, IL-22, IL-1β, LCN-2, and CXCL-2 in auricle) observed in the rhIL-23 group was confirmed in the mice administered with AJP001 conjugated peptide (SEQ ID NO: 6).

[Example 8] Monkey immunogenicity evaluation test using AJP001 conjugated peptide (SEQ ID NO: 6)

AJP001 conjugated peptide (SEQ ID NO: 6) was dissolved in physiological saline and subcutaneously administered to Macaca fascicularis (female, 4-5-week-old, N=4) at 5 mg/body 3 times at two-week intervals. Blood was collected every two weeks from the first day of administration until week 16, and the antibody titer (GMT) and binding to IL-23 of the serum at each time point were measured by the ELISA method (same method as in Example 1; the secondary antibody used was Anti-Monkey IgG (Abcam)). The results are shown in Fig. 7. In Fig. 7, "*" means P<0.05.
As shown in Fig. 7 (a), the antibody titer increased from 2 weeks after the initial administration and persisted until 16 weeks. In addition, the monkey antiserum 6 weeks after the initial administration showed binding ability to rhIL-23 but binding to rhIL-23 was not observed (Fig. 7 (b)).
Then, IgG was purified using Protein G HP spin Trap (GE Healthcare) from the monkey antiserum obtained 6 weeks after the initial administration, and the action on IL-17 production when mouse splenocytes were stimulated with rhIL-23 was evaluated (same method as in Example 5). The results are shown in Fig. 7(c).

As shown in Fig. 7(c), IL-17 production was suppressed by adding 300 ug/mL of IgG derived from monkey antiserum.

Furthermore, epitope analysis was performed using monkey antiserum obtained with AJP001 conjugated peptide (SEQ ID NO: 6). A 96-well plate with AJP001 conjugated peptide (SEQ ID NO: 6) immobilized was blocked with goat serum. AJP001 conjugated peptide (SEQ ID NOs: 6 to 14) was added at 100 $\mu$g/mL to monkey serum derived from AJP001 conjugated peptide (SEQ ID NO: 6), and reacted overnight at 4°C. The reaction solution was added to the immobilized plate and allowed to stand overnight at 4°C. In addition, a control well to which only monkey serum derived from AJP001 conjugated peptide (SEQ ID NO: 6) was added and an untreated well to which no serum was added were provided. After washing the wells with PBS-T, Anti-Monkey IgG antibody (Abeam) diluted with 5% skim milk/PBS was added and the mixture was shaken at room temperature for 3 hr. After washing the wells with PBS-T, TMB solution was added, the wells were allowed to stand for 30 min in the dark, the reaction was stopped by adding 0.5M $H_2SO_4$, and the absorbance at 450 nm was measured using a plate reader. The binding ratio was calculated by the following formula and using the absorbance values of control well (A), untreated (B), and monkey serum (C) incubated with AJP001 conjugated peptide (SEQ ID NOs: 6 to 14).

$$\texttt{binding ratio (\%) = 100 - [100 × (C-B/A-B)]}$$

The results are shown in Fig. 7(d). As shown in Fig. 7(d), the antiserum of AJP001 conjugated peptide (SEQ ID NO: 6) bound to AJP001 conjugated peptide (SEQ ID NO: 6-11), but the binding rate to AJP001 conjugated peptide (SEQ ID NO: 12-14) decreased. The results suggest that the antibody induced by AJP001 conjugated peptide (SEQ ID NO: 6) recognizes the C-terminal region in the B cell antigen sequence.

[Example 9] Efficacy evaluation test using AJP001 conjugated peptide (SEQ ID NO: 2) in 2,4,6-trinitrobenzenesulfonic acid (TNBS)-induced mouse colitis model

AJP001 conjugated peptide (SEQ ID NO: 2) was dissolved in physiological saline, mixed with an equal amount of 1 mg/mL Type-B/K CpG-ODN K3 (GeneDesign, Inc., hereinafter sometimes to be referred to as "CpG"), and subcutaneously administered to BALB/c mouse (female, 6-week-old, N=10) at 1 mg/body 3 times at two-week intervals. At the time point of 6 weeks after the initial administration, TNBS dissolved in 30% ethanol was administered by intestinal infusion at 2 mg/body. With the first administration of TNBS as Day 0, TNBS was administered by intestinal infusion at 3 mg/body on Day 7 to induce colitis. A Normal group and a 30% EtOH group, each without administration of TNBS, a Saline/TNBS group with administration of TNBS but without administration of the peptide, and a CpG/TNBS group with administration of only an adjuvant were provided. Body weight was measured on Days 1, 2, 3, 4, 7, 8, 9, and 10, including the day of TNBS administration. After euthanasia by cardiac blood collection under deep anesthesia on Day 10, the obtained serum was measured for the antibody titer (GMT) and binding to mIL-23 by the ELISA method (same method as in Example 1). In addition, large intestinal tissue was collected, the length (cm) of the large intestine was measured, a 2 cm region from the anus was subjected to the measurement of cytokine mRNA expression, and a 2 to 4 cm region was subjected to histopathological examination. As to the expression of cytokine mRNA, the region was bead disrupted under ice-cooling, total RNA was extracted using RNeasy Fibrous Tissue Kit (Qiagen), and then cDNA of the extracted RNA was prepared using High-Capacity cDNA Reverse Transcription Kit (Applied Biomedical Inc). Using TaqMan Gene Expression Assays (GAPDH:Mm99999915_g1; IL-17A:Mm00439618_m1; IL-17F:Mm00521423_m1; IL-22:Mm01226722_m1; IL-23:Mm00518984_m1; IL-1$\beta$:Mm00434228_m1), Ct value was measured using Applied Biosystems 7900HT Fast Real Time PCR System. The mRNA expression level was calculated by the $\Delta\Delta$Ct method and evaluated as a relative value to the normal group. For histopathological examination, the collected large intestine was fixed with 10% neutral formalin, and then a specimen was prepared according to a conventional method and stained with HE. The HE-stained specimen was observed under a microscope, scored for inflammatory cell infiltration (Grade: 0-3), depth of colorectal tissue damage (Grade: 0-3), and range of colorectal mucosal damage (Grade: 0-4), and the total score was calculated.

The results of efficacy evaluation in the antibody titer of AJP001 conjugated peptide (SEQ ID NO: 2), binding to rmIL-23, weight change (change rate from Day 0), length of large intestine, mRNA expression in colon tissue, and histopathological examination are shown in Fig. 8-1, Fig. 8-2, Fig. 8-3, and Fig. 8-4. In the Figures, "#" means that it is significant (P<0.05) compared with "0w". "*" means that it is significant (P<0.05) compared with "Saline/TNBS" or "CpG/TNBS".

As shown in the Figures, a suppressive action on colitis observed in the Saline/TNBS or CpG/TNBS groups (body weight loss, shortened large intestine length, inflammatory changes in large intestine tissues, and mRNA expression of IL-17A, IL-22, and IL-1$\beta$ in tissues) was confirmed in the mice administered with AJP001 conjugated peptide (SEQ ID NO: 2).

[Industrial Applicability]

According to the present invention, a peptide vaccine for the treatment and/or prevention of various diseases related to IL-23 can be produced at a low cost. Therefore, the present invention is extremely useful in the field of manufacture of pharmaceutical products.

This application is based on a patent application No. 2021-199561 filed in Japan (filing date: December 8, 2021) and a patent application No. 2022-162795 filed in Japan (filing date: October 7, 2022), the contents of which are incorporated in full herein.

**Claims**

1. A vaccine composition comprising a complex of a T cell receptor antigen peptide and a B cell receptor antigen peptide and capable of inducing the production of an antibody against IL-23, wherein the B cell receptor antigen peptide is represented by the following formula (I):

$$X1-X2-X3-X4-X5-X6-X7-X8 \qquad (I)$$

wherein

X1 is S, A, G, T, K, or R,
X2 is P, A, G, S, T, K, or R,
X3 is S, A, G, T, K, or R,
X4 is Q, A, G, T, or N,
X5 is P, A, G, S, T, Q, or N,
X6 is W, A, Y, or F,
X7 is Q, A, G, T, or N, and
X8 is R, A, G, or K.

2. The vaccine composition according to claim 1, wherein X6 in the B cell receptor antigen peptide is W.

3. The vaccine composition according to claim 1 or 2, wherein X7 in the B cell receptor antigen peptide is Q.

4. The vaccine composition according to any one of claims 1 to 3, wherein X8 in the B cell receptor antigen peptide is R.

5. The vaccine composition according to any one of claims 1 to 4, wherein the B cell receptor antigen peptide comprises the amino acid sequence shown in any of SEQ ID NOs: 2, 6 - 14 and 17 - 26.

6. The vaccine composition according to any one of claims 1 to 5, wherein the T cell receptor antigen peptide comprises the amino acid sequence shown in SEQ ID NO: 38.

7. The vaccine composition according to any one of claims 1 to 6, wherein, in the complex, the C-terminus of the T cell receptor antigen peptide is bonded to the N-terminus of the B cell receptor antigen peptide.

8. The vaccine composition according to any one of claims 1 to 7, wherein the T cell receptor antigen peptide and the B cell receptor antigen peptide are bonded via a linker.

9. The vaccine composition according to any one of claims 1 to 8, which does not contain an additive as an adjuvant.

10. The vaccine composition according to any one of claims 1 to 9, for the treatment or prevention of an IL-23-associated disease.

11. The vaccine composition according to claim 10, wherein the IL-23-associated disease is selected from the group consisting of psoriasis, psoriatic arthritis, rheumatoid arthritis, systemic lupus erythematosus, diabetes (preferably type I diabetes), atherosclerosis, inflammatory bowel disease (IBD)/Crohn's disease, multiple sclerosis, Behcet's disease, ankylopoietic spondylarthritis, Vogt-Koyanagi-Harada disease, chronic granulomatous disease, hidradenitis suppurativa, Anti-Neutrophil Cytoplasmic Antibody (ANCA) Associated Vasculitis, neurodegenerative disease, atopic dermatitis, graft-versus-host disease, and cancer.

[Fig. 1]

(a) Mouse IL-23

AJP001 (helper T cell epitope)-Ahx-B cell epitope

(b) Antibody titer

(c) Binding to rmIL-23

[Fig. 2]

**(a) Antibody titer**

**(b) Binding to rmIL-23**

**(c) ELISPOT (IFN-γ)**

**(d) ELISPOT (IL-4)**

[Fig. 3]

(a) Antibody titer (6w)

(b) Ear swelling

(c) Histology

Normal        2% IMQ        SEQ ID NO:2

(d) Inflammatory cell □Edema □Hypertrophy

[Fig. 4]

(a) Ear swelling

(b) inflammatory cytokine expression

IL-17A

IL-22

IL-1β

LCN-2

CXCL-2

[Fig. 5]

(a) Antibody titer

(b) Binding to rhIL-23

(c) ELISPOT (IFN-γ)

(d) ELISPOT (IL-4)

[Fig. 6]

**(a) Ear swelling**

**(b) Inflammatory cytokine mRNA expression**

[Fig. 7]

(a) Antibody titer

(b) Binding to rIL-23

(c) Neutralization activity (Monkey purified IgG)

(d) Epitope mapping (Monkey antisera)

[Fig. 8-1]

(a) Antibody titer

(b) mIL-23 Binding

(c) Body weight

[Fig. 8-2]

# (d) Colon length (cm)

Colon Length (cm)

[Fig. 8-3]

(e) Inflammatory cytokine mRNA expression

EP 4 445 910 A1

[Fig. 8-4]

## (f) Histology

## INTERNATIONAL SEARCH REPORT

| International application No. |
|---|
| **PCT/JP2022/045045** |

### A. CLASSIFICATION OF SUBJECT MATTER

*A61K 39/00*(2006.01)i; *A61K 47/55*(2017.01)i; *A61P 1/04*(2006.01)i; *A61P 3/10*(2006.01)i; *A61P 9/00*(2006.01)i; *A61P 9/10*(2006.01)i; *A61P 17/00*(2006.01)i; *A61P 17/06*(2006.01)i; *A61P 19/00*(2006.01)i; *A61P 19/02*(2006.01)i; *A61P 25/00*(2006.01)i; *A61P 25/28*(2006.01)i; *A61P 27/02*(2006.01)i; *A61P 29/00*(2006.01)i; *A61P 35/00*(2006.01)i; *A61P 37/00*(2006.01)i; *A61P 37/02*(2006.01)i; *A61P 37/04*(2006.01)i; *A61P 37/06*(2006.01)i; *A61P 37/08*(2006.01)i; *A61P 43/00*(2006.01)i; *C07K 7/06*(2006.01)i; *C07K 7/08*(2006.01)i; *C07K 14/00*(2006.01)i; *C07K 14/54*(2006.01)i
FI:  A61K39/00 H; A61P37/04; A61K47/55; A61P17/06; A61P19/02; A61P37/00; A61P29/00 101; A61P3/10; A61P9/10; A61P35/00; A61P27/02; A61P9/00; A61P29/00; A61P19/00; A61P17/00; A61P37/06; A61P25/28; A61P43/00 105; A61P1/04; A61P37/08; A61P37/02; A61P25/00; C07K7/08; C07K14/00; C07K14/54 ZNA; C07K7/06 ZNA

According to International Patent Classification (IPC) or to both national classification and IPC

### B. FIELDS SEARCHED

Minimum documentation searched (classification system followed by classification symbols)

A61K39/00; A61K47/55; A61P1/04; A61P3/10; A61P9/00; A61P9/10; A61P17/00; A61P17/06; A61P19/00; A61P19/02; A61P25/00; A61P25/28; A61P27/02; A61P29/00; A61P35/00; A61P37/00; A61P37/02; A61P37/04; A61P37/06; A61P37/08; A61P43/00; C07K7/06; C07K7/08; C07K14/00; C07K14/54

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| Y | WO 2017/164409 A1 (OSAKA UNIV.) 28 September 2017 (2017-09-28) whole document, particularly, paragraphs [0001], [0010], [0011], [0018]-[0020], [0030], [0031], [0033], [0034], [0040], [0070]-[0081], fig. 9-22 | 1-11 |
| Y | JP 2010-518858 A (SCHERING CORP.) 03 June 2010 (2010-06-03) whole document, particularly, paragraphs [0001], [0019], [0026], [0085]-[0087], [0157], [0158], [0186]-[0198] | 1-11 |

✓ Further documents are listed in the continuation of Box C.    ✓ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **13 February 2023** | **21 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**

| International application No. |
|---|
| **PCT/JP2022/045045** |

**C.** **DOCUMENTS CONSIDERED TO BE RELEVANT**

| Category* | Citation of document, with indication, where appropriate, of the relevant passages | Relevant to claim No. |
|---|---|---|
| Y | KATAYAMA, H. Development of psoriasis by continuous neutrophil infiltration into the epidermis. Experimental Dermatology. 14 August 2018, vol. 27, no. 10, pages 1084-1091, doi:10.1111/exd.13746<br>abstract, page 1084, left column, line 2 to page 1085, left column, line 5, page 1088, left column, line 12 to page 1088, right column, line 15, page 1089, right column, lines 15-7 from the bottom | 1-11 |
| Y | TENMA, A. et al. AJP001, a novel helper T-cell epitope, induces a humoral immune response with activation of innate immunity when included in a peptide vaccine. FASEB BioAdvances. 22 November 2019, vol. 1, no. 12, pages 760-772, doi:10.1096/fba.2019-00056<br>abstract, table 1, fig. 2, 6 | 1-11 |
| Y | 株式会社ファンペップ, 2020年12月期 決算説明会, 18 February 2021, slide no. 1-53, https://fs2.magicalir.net/tdnet/2021/4881/20210218466415.pdf, (FUNPEP CO., LTD.), non-official translation (Financial Results Briefing on December 2020.)<br>slide no. 5, 15, 16, 33, 43, 51, 52 | 1-11 |

Form PCT/ISA/210 (second sheet) (January 2015)

36

**INTERNATIONAL SEARCH REPORT**

International application No.

**PCT/JP2022/045045**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "The form of Annex C/ST.25 text file" above shall read as "the form of ST.26.".

Form PCT/ISA/210 (continuation of first sheet) (January 2015)

INTERNATIONAL SEARCH REPORT
Information on patent family members

International application No.

**PCT/JP2022/045045**

| Patent document cited in search report | | | Publication date (day/month/year) | Patent family member(s) | Publication date (day/month/year) |
|---|---|---|---|---|---|
| WO | 2017/164409 | A1 | 28 September 2017 | US 2019/0105388 A1 paragraphs [0001], [0020]-[0033], [0043]-[0046], [0061], [0063], [0064], [0070], [0102]-[0117]<br>EP 3434279 A1 | |
| JP | 2010-518858 | A | 03 June 2010 | US 2010/0111966 A1 paragraphs [0001], [0018], [0025], [0085]-[0087], [0153], [0154], [0184]-[0194], fig. 9-22<br>EP 2064242 A1<br>WO 2008/103473 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016193405 A
- WO 2016047763 A
- US 9102752 B
- JP 2021199561 A
- JP DECEMBER82021 B
- JP 2022162795 A

**Non-patent literature cited in the description**

- *WHO report,* 2016
- **CHRISTINA H LIU et al.** *Nat Immunol.,* 18 October 2017, vol. 18 (11), 1175-1180
- **YOICHIRO IWAKURA et al.** *J Clin Invest.,* May 2006, vol. 116 (5), 1218-22
- **SARAH L GAFFEN et al.** *Nat Rev Immunol.,* September 2014, vol. 14 (9), 585-600
- **ROJO A RATSIMANDRESY et al.** *Vaccine,* 2011, vol. 29, 9329-9336
- **QINGDONG GUAN et al.** *Immunotherapy,* 2013, vol. 5, 1313-1322
- Synthesis of Organic Compounds IV. Jikken Kagaku Kouza. vol. 16